# EUROPEAN PATENT SPECIFICATION

(11) **EP 2 321 332 B1**
(45) Date of publication and mention of the grant of the patent: **27.03.2013**
(21) Application number: 09808504.6
(22) Date of filing: 19.08.2009
(51) Int. Cl.: C07H 21/04, A61K 31/70, C12P 19/34, C12Q 1/68

(54) **SELF-AVOIDING MOLECULAR RECOGNITION SYSTEMS IN DNA AMPLIFICATION**
SELBSTVERMEIDENDE MOLEKULARE ERKENNUNGSSYSTEME IN DNA-AMPLIFIKATIONEN
SYSTÈMES DE RECONNAISSANCE MOLÉCULAIRE À ÉVITEMENT AUTOMATIQUE DANS UNE AMPLIFICATION D'ADN

(30) Priority: 20.08.2008 US 229159
(43) Date of publication of application: 18.05.2011
(73) Proprietor: Benner, Steven A., Gainsville, FL 32605-4147 (US); Hoshika, Shuichi, Gainesville, FL 32601 (US); Chen, Fei, Gainesville, FL 32601 (US)
(72) Inventor: Benner, Steven A., Gainsville, FL 32605-4147 (US); Hoshika, Shuichi, Gainesville, FL 32601 (US); Chen, Fei, Gainesville, FL 32601 (US)
(74) Representative: Gallois, Valérie
(86) International application number: PCT/US2009/004718
(87) International publication number: WO 2010/021702

(56) References cited:
- WO-A1-2006/095981
- US-A- 5 432 272
- US-A1- 2008 090 235
- US-A1- 2008 146 787
- US-B2- 7 371 580
- WOO J ET AL: "G/C-MODIFIED OLIGODEOXYNUCLEOTIDES WITH SELECTIVE COMPLEMENTARITY: SYNTHESIS AND HYBRIDIZATION PROPERTIES", NUCLEIC ACIDS RESEARCH, OXFORD UNIVERSITY PRESS, SURREY, GB, vol. 24, no. 13, 1 July 1996 (1996-07-01), pages 2470-2475, XP000621694, ISSN: 0305-1048, DOI: 10.1093/NAR/24.13.2470
- KUTYAVIN I V ET AL: "Oligonucleotides containing 2-aminoadenine and 2-thiothymine act as selectively binding complementary agents", BIOCHEMISTRY, AMERICAN CHEMICAL SOCIETY, US, vol. 35, no. 34, 1 January 1996 (1996-01-01), pages 11170-11176, XP002217382, ISSN: 0006-2960, DOI: 10.1021/BI960626V
- HOSHIKA SHUICHI ET AL: "Self-Avoiding Molecular Recognition Systems (SAMRS).", NUCLEIC ACIDS SYMPOSIUM SERIES (2004) 2008 LNKD- PUBMED:18776287, no. 52, 2008, pages 129-130, XP000002658214, ISSN: 1746-8272
- HOSHIKA SHUICHI ET AL: "Artificial genetic systems: self-avoiding DNA in PCR and multiplexed PCR.", ANGEWANDTE CHEMIE (INTERNATIONAL ED. IN ENGLISH) 26 JUL 2010 LNKD- PUBMED:20586087, vol. 49, no. 32, 26 July 2010 (2010-07-26) , pages 5554-5557, XP000002658215, ISSN: 1521-3773
- AHLBORN ET AL.: 'Isostable DNA.' J. AM. CHEM. SOC. vol. 129, no. 49, 2007, pages 15218 - 15232, XP008142767

## Description

### Background of the Invention

### 1. Field of the Invention

This invention relates to the field of nucleic acid chemistry, more specifically to the field of compositions of matter that serve as primers for the copying of DNA and RNA, and more specifically to the amplification by the polymerase chain reaction of DNA. These compositions of matter include non-standard nucleotides that bind to natural complements to contribute to the stability of duplexes following rules, where those rules are delimited by a large number of experimental data, do not bind as strongly to themselves, and are accepted by DNA polymerases in both primers and templates discovered through experimentation.

### 2. Description of Related Art

Over the past 15 years scientists have sought innovative molecular recognition systems that have binding properties that are useful in different ways. The structures of some of these systems have been modeled along the lines of DNA and RNA. Further, as with DNA and RNA, the molecular recognition systems have been useful because they bind to other components of the molecular recognition systems and/or to natural DNA and RNA following rules that can be expressed in a form that guides practitioners of ordinary skill in the art and enables them to do useful things.

DNA serves as an archetype to illustrate both molecular structure and rule base recognition. As it is built from four building blocks, the number of different DNA sequences of length n (= 4*ⁿ*) that would fall within a patent for the DNA molecular recognition system would be enormous. Accordingly, such a system would be considered patentable only if heuristic rules were available that allows one skilled in the art to predict what DNA sequences bind to what other sequences without undue experimentation.

Such heuristic rules have come for natural DNA, RNA, and a variety of its modified forms (e.g., 2'-OMe modified RNA) and analogs (e.g. PNA) by performing substantial numbers of melting temperature experiments. In general, without these, molecular recognition cannot be predicted in a useful way. For DNA, these experiments have been done for many years and have yielded many examples of heuristic rules. Three rules are especially important:A pairs with T, G pairs with C, and the strands are antiparallel. Documented now for several decade, these rules permit one skilled in the art to design of two DNA molecules that bind to each other in aqueous solution. When the rules are perfectly followed, two perfectly complementary DNA strands of a substantial length (15-20 nucleotides is normally sufficient in physiological buffers at 37 °C) will bind to each other with substantial selectivity even in complex mixtures containing many other DNA molecules.

Further rules have been developed over the years to permit the prediction of general trends in DNA:DNA binding affinity. longer DNA strands generally bind to their partners with higher melting temperatures (Tₘs) than shorter strands. G:C pairs generally contribute more to duplex stability than A:T pairs. These rules are not absolutely necessary for a skilled practitioner to obtain utility from the DNA system. More highly parameterized models improve on the estimates of melting temperatures [All98a] [All98b] [Mar85] [Mat98], but are not necessary for much utility in the field. Thus, the kinds of rules that one obtains from ca. 100 melting temperature experiments are necessary and sufficient to allowing a molecular recognition system modeled on DNA to have a core level of utility; the kinds of rules obtained from thousands of melting temperature experiments are not necessary for the system to have a core utility, although they may expand the utility of the system.

One class of unnatural (or, as used here, "non-standard") DNA analog expands the number of nucleobase pairs by adding nucleotides (for example, X and Y) that bind to each other (here, to form an X:Y pair) and contribute to overall duplex stability, but where X and Y, when matched with standard DNA nucleotides, do not contribute to the duplex stability. In this illustration, the most useful genetic alphabet expanded by adding X and Y has biophysical properties where A:P, T:P, G:P, C:P, A:X, T:X, G:X, and C:X "mismatched" pairs all contribute to duplex stability less than the X:Y pair. Still more useful are when the mismatched pairs contribute to duplex stability about the same as mismatches between standard pairs (e.g., A:G, C:A, T:G, and so on) or a mismatch between a standard nucleotide and an abasic site, with the X:Y pair contributing more than these mismatches.

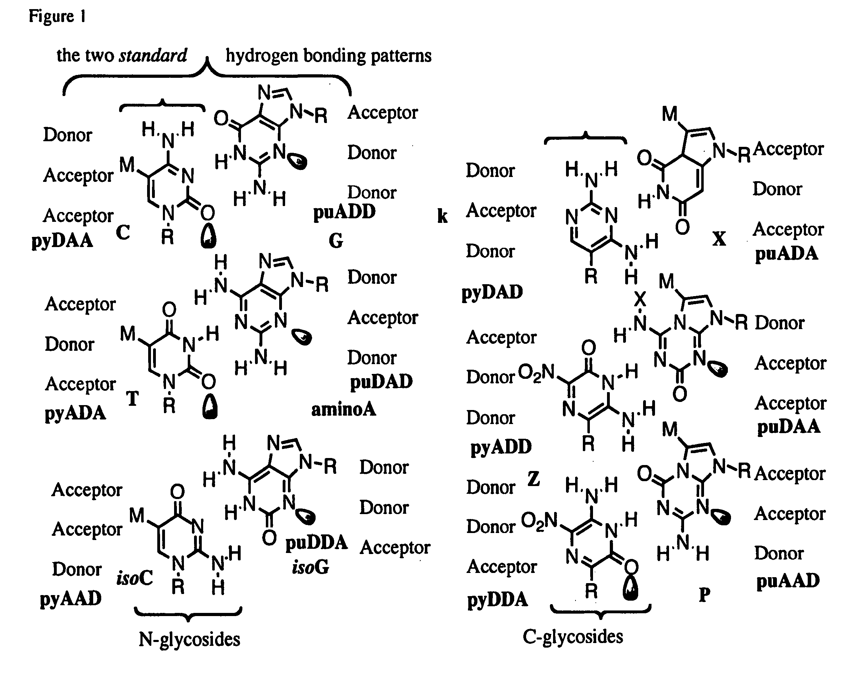

An archetype of a human-invented rule-based molecular recognition system of this type was disclosed in US Patent 5432272 and its successors (Figure 1). Here, the design of this artificial molecular recognition system began with the observation that two principles of complementarity govern the Watson-Crick pairing of nucleic acids: *size complementarity* (large purines pair with small pyrimidines) and *hydrogen bonding complementarity* (hydrogen bond donors from one nucleobase pair with hydrogen bond acceptors from the other). These two principles give rise to the simple rules for base pairing ("A pairs with T, G pairs with C") that underlie genetics, molecular biology, and biotechnology. US Patent 5432272 pointed out that these principles can be met by nucleotides other than adenine (A) and thymine (T), and guanine (G) and cytosine (C). Rather, twelve nucleobases forming six base pairs joined by mutually exclusive hydrogen bonding patterns might be possible within the geometry of the Watson-Crick base pair. Figure 1 shows some of the standard and non-standard nucleobase pairs, together with the nomenclature to designate them. Those nucleobase analogs presenting non-standard hydrogen bonding patterns are part of an Artificially Expanded Genetic Information System, or AEGIS.

US Patent 5432272 and its successors taught that the hydrogen bonding pattern that makes an AEGIS component useful as a unit of molecular recognition can be discussed independent of the heterocycle that implements it. This means that different heterocycles can often serve interchangeably as molecular recognition elements. This, in turn, permits the elements of an artificial molecular recognition system to be chosen based on considerations other than simple recognition. Thus, the pyADA hydrogen bonding pattern in AEGIS is implemented by thymidine, uridine, uridine derivatives carrying a 5-position linker attached to a fluorescent moiety, uridine derivatives carrying a 5-position linker attached to a biotin, and pseudouridine, for example.

After ca. 100 melting temperature experiments, molecular recognition within the AEGIS system was adequate to support utility. An archetypal application of AEGIS is in the branched DNA (bDNA) assay used to measure levels of HIV, hepatitis B, and hepatitis C viruses in human patients [Elb04a][Elb04b]. As this example shows, even though the behavior of DNA duplexes built from AEGIS components having different sequences are not identical and may not be precisely predictable, this has not prevented the AEGIS from improving the care of some 400,000 patients annually [Ben04]. It also illustrates the utility of *orthogonality* in the analytical chemistry of nucleic acids, a utility that is exploited only when these are used in mixtures of two or more molecules with different sequences. In oligonucleotides built to contain orthogonal molecular recognition elements like AEGIS components, members of the system bind well only to other oligonucleotides that contain AEGIS components, and do not bind to natural DNA sequences.

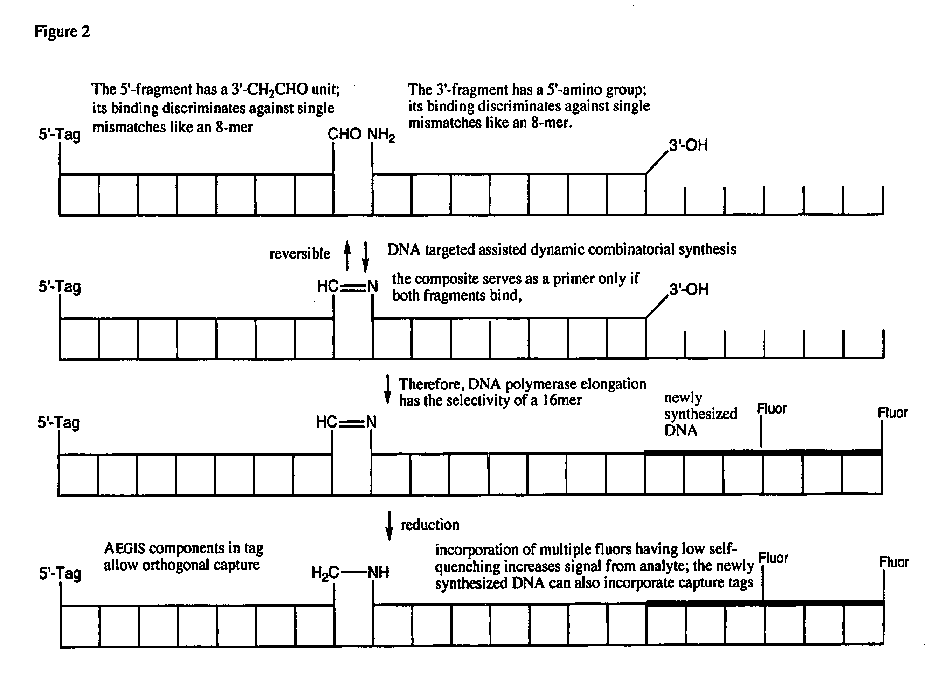

It has long been recognized in the literature that an alternative type of DNA analogs having opposite behavior might have a different type of utility. Here, the nucleotide analogs are "self-avoiding". Self-avoiding nucleotide building blocks are designated A*, T*, G*, and C*. To be "self-avoiding", the A*:T, T*:A, G*:C and C*:G pairs all must contribute to duplex stability more than standard mismatches (or mismatches to abasic sites), but where the A*:T* and G*:C* pairs, formally matched, contribute to duplex stability less. The utility of this property in a DNA-like structure was recognized as long ago as 15 years, where Gamper and others sought to incorporate versions of A* and T* (where A* was diaminopurine and T* was 2-thiothymidine) into a single DNA or RNA molecule to prevent if from forming "secondary structure", where parts of the same molecule form Watson-Crick duplexes [Kut96]. They devoted nearly a decade of work developing a corresponding G* and C*, with inosine being preferred as G*. For C*, their preferred choices included a 5-6 fused pyrrolopyrimidine system (Figure 2) [Woo96], a deaminated analog of cytidine related to the natural product zebularine [Gam06]][Lahoud et al. (2008) Properties of pseudo-complementary DNA substituted with weakly pairing analogs of guanine or cytosine. Nucleic Acids Res. 36, 6999-7008], and 4-N alkylated derivatives of cytosine [Lah08a,b,c]. Must of their work has been devoted to obtaining polymerases that add these self-avoiding nucleotides to the 3'-end of a primer using template directed incorporation of diaminopurine, 2-thiothymidine, and inosine deoxynucleoside triphosphates.

The first intellectual property to emerge from this work was disclosed by US patent 5912340 (issued 1999/06/15). US5912340 was not concerned with creating primers for DNA polymerases or multiplexed PCR. Rather, US5912340 claimed a pair of oligonucleotides (ODNs) that were formally complementary to each other, that could bind to their complement if built from natural nucleotides, but did not bind to each other. This pair would be useful to invade duplex DNA.

The inventors of US5912340 were satisfied if "sufficient" numbers of their nucleotides (analogous to the * analogs discussed here) were incorporated to prevent two oligonucleotides in the pair from binding to each other, or (in later work) if sufficient numbers of the analogs were present to prevent the DNA or RNA molecule from folding on itself. US5912340 did not provide any melting temperatures, nor did subsequent work, nor did it provide assurance that one of ordinary skill in the art could get useful predictability (without undue experimentation) from oligonucleotides built from the components that they (and they and others in subsequent work) provided. Further, as US5912340 provided no data with polymerases acting on these unnatural compounds as templates or primers, it was not certain that they would be accepted by polymerases, and it was definitively uncertain whether they would be accepted by polymerases with sufficient efficiency to support the demands of PCR.

Nor was it necessary for US5912340 or subsequent work to do so, as its principal goal was to obtain formally complementary pairs that did not bind to each other. It did not claim to provide primers, let alone primers suitable for PCR.

More recently, efforts to use nucleotides containing diaminopurine, 2-thiothymidine, inosine, and pyrrolopyrimidine in a set of probes were described in the patent literature (US Patent 7371580, published as US 2003/0211474 A1 on 2009/11/15, and issued on 2008/05/13). Here, the utility proposed was to prevent self-association of these probes from creating what these specifications called "cross-binding" between the probes, while still allowing the probes containing these * nucleotide analogs to bind to natural DNA. This patent, however, contained very little experimental data, including few melting temperatures, inadequate to provide guidance as to how to use these systems, and no working example of these probes in highly multiplexed combination.

If it could be workable, a particularly valuable application of a self-avoiding molecular recognition system (SAMRS) would be in multiplexed polymerase chain reaction (PCR) architectures. Here, oligonucleotides built from * SAMRS nucleotides would be prepared in pairs, where the members of the pairs would not serve as probes, but rather as PCR primers. One member of the pair would serve as a forward primer, being complementary to a target oligonucleotide; the other member of the pair would be identical to a segment of the target nucleotide that lies in the 5'-direction from the position where the first member of the pair is complementary. Such pairs, if built from SAMRS nucleotides, could be presented in plurality (more than one pair), or even in large abundance. By being self-avoiding, they should be able to support the PCR amplification of many targets at the same time in a single reaction tube and offer a solution to the "multiplexed PCR problem".

A solution to the multiplexed PCR problem is a "Holy Grail" in the analytical chemistry of DNA. Evidence for this includes the literature descriptions of multiple attempts by many research groups to achieve it [Bro97], often with clever architectures, including cyclization to remove the "PCR mess" [Fre07] that arises from cross-binding and off-target priming of primer pairs. As another example, in parallel genome sequencing, the multiplexed PCR problem is avoided by first diluting a complex mixture of DNA molecules to a single molecule state, and then amplifying single molecules separately. This does not solve the multiplexed PCR problem, but rather avoids it, at the cost of dealing with all of the problems that arise from single molecule chemistry.

US2008/090235 discloses amplification methods using primers with artificial nucleotides.

Despite the well-known multiplexed PCR problem, US 7371580 does not mention that its nucleotide analogs might be used to solve it. Experiments now suggest (see below) that many of the compositions of matter taught in this and other prior art do not work to a useful extent in pairs of primers in PCR reactions, and that one of skill in the art cannot, without undue experimentation, apply these systems for any obvious utility. It is the purpose of the instant invention to provide self-avoiding nucleotide analogs together with a sufficient amount of experimental data to allow an understanding of how they might be used, especially in combination, and especially in PCR, including multiplexed PCR, as well as kits that contain them, as well as processes that use them.

### (g) Brief Summary of the Invention

The scope of the invention is defined by the claims and any information that does not fall within the claims is provided for information only.

This inventors identified SAMRS nucleobase analogs (T*, A*, G* and C*) that, when paired against natural adenine, thymine, cytosine, and guanine (respectively) add to duplex stability, when paired against A*, T*, G* and C* (respectively) subtract from duplex stability, and where an oligonucleotide analog containing SAMRS nucleobases near the 3'-end can serve as a primer able to support PCR amplifications. Further, this description provides for specific compositions for those oligonucleotide analogs that serve as prime pairs in a polymerase chain reaction (PCR), and provides experimental evidence that these compositions can so serve. Further, this description provides for compositions of matter that comprise a plurality of pairs of these analogs that support multiplexed PCR, and provides experimental evidence that these compositions can support multiplexed PCR at concentrations that are high enough to support useful multiplexed PCR.

### (h) Brief Description of the Drawings

**Fig. 1**. The AEGIS system. 12 nucleobases in a nucleic acid alphabet that form specific pairs with the constraints of the Watson-Crick geometry. These make an artificially expanded genetic information system (AEGIS) to be applied to tools for systems biology. Pyrimidine base analogs are designated "py", purine by "pu". Upper case letters following a designation indicate the hydrogen bonding pattern of acceptor (A) and donor (D) groups. Thus, cytosine is pyDAA. Note letter designations (Z and P), orbitals holding minor groove unshared electron pairs (shaded lobes; these may be recognition elements for some polymerases), and positions where tags can be appended (M).
**Fig. 2****.** SNAP2 architecture for primers that dynamically assemble on a template from two fragments, each 8 nucleotides in length, one terminated with 3'-CH₂CHO (on the 5'-DNA fragment), the other with a 5'-NH₂ (on the 3'-DNA fragment). These reversibly form a composite, joined via an imine linker under conditions of dynamic equilibrium. Imine formation is reversible in water, and reversibility ensures that the tightest binding complement perfectly matched to the template is formed. Should this composite prime synthesis of DNA using a DNA polymerase, the specificity of priming should be characteristic of a 16-mer (and therefore unique in the human genome), as both sequences must be adjacent on the template for priming to occur. The discrimination against mismatches, however, should be that characteristic of an 8-mer, and therefore be very high. This architecture has a superficial resemblance to one proposed by Studier [Stu89], Szybalski, [Szy90], Kotler et al. [Kot93] and others, where multiple short fragments are ligated as part of a sequencing architecture. In these proposals, the covalent bonding forming step is irreversible, and therefore does not benefit from the features of a dynamic equilibrium. For a description of preliminary data, see [Lea06].

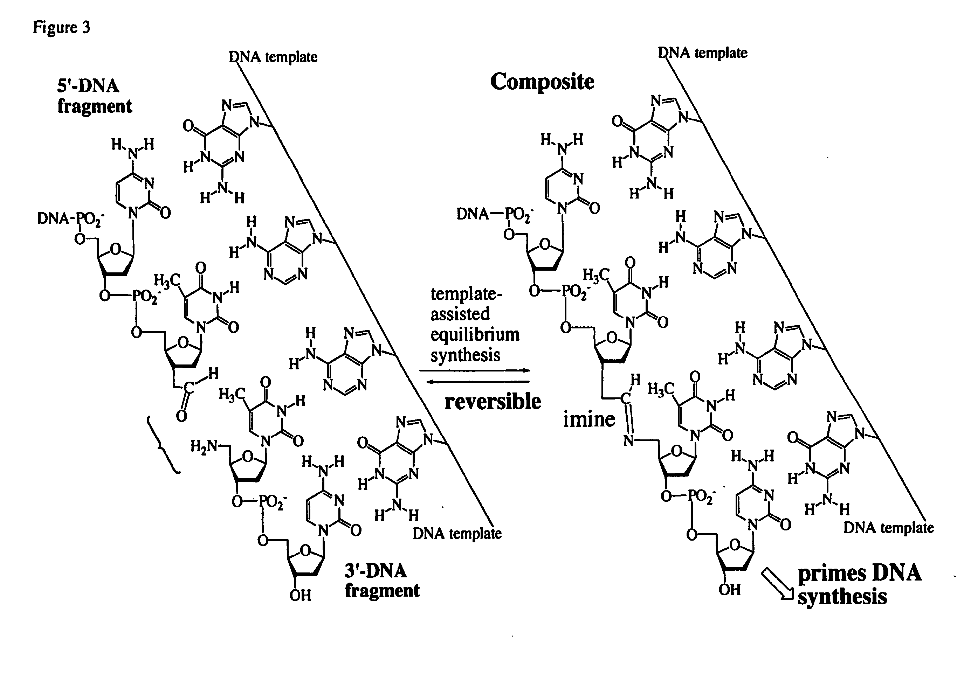

**Fig. 3****.** Details of the chemical implementation of the architecture shown in Fig. 2. Here, an imine linkage is transiently formed from the two primer fragments. For preliminary data, see [Lea06]

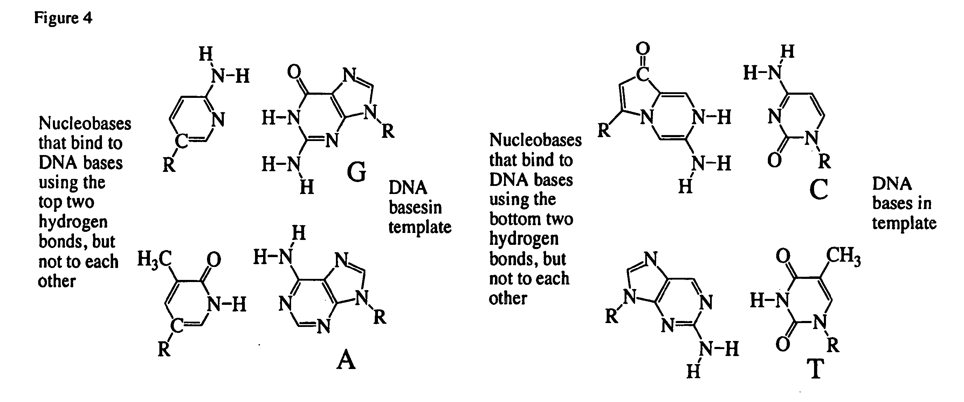

**Fig. 4****.** Heterocycles implementing the Self-Avoiding Molecular Recognition System (SAMRS), with the teaching that by using base pairs joined by two hydrogen bonds, a series ofnucleobases can be designed, to be placed on a PNA or DNA backbone, that will bind to natural G, A, C, and T (by two hydrogen bonds), but not to each other (by more than one hydrogen bond). This allows the sequences in the fragments to be orthogonal to other fragment sequences.

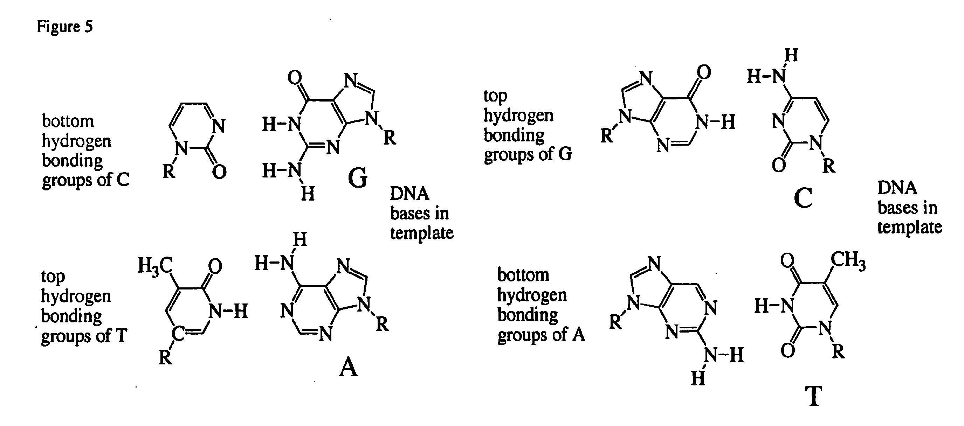

**Fig. 5****.** Heterocycles implementing the Self-Avoiding Molecular Recognition System (SAMRS).

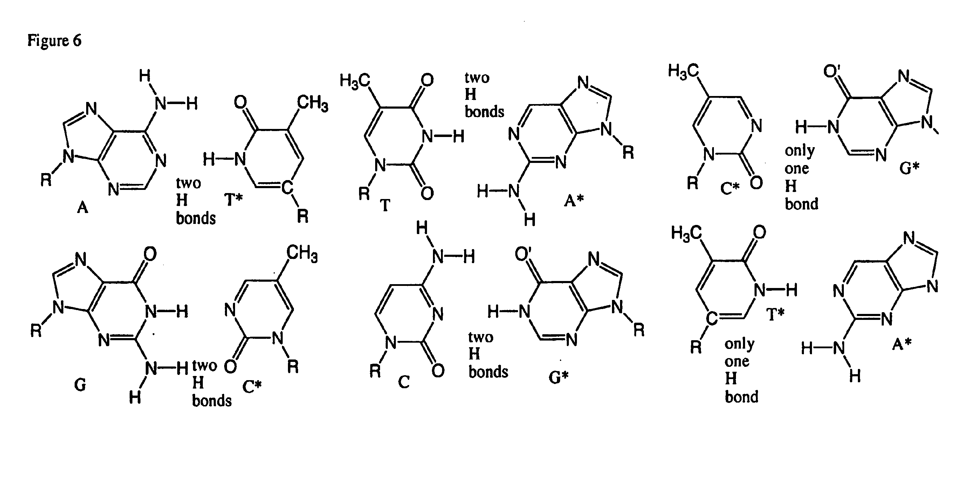

**Fig. 6****.** Heterocycles implementing the Self-Avoiding Molecular Recognition System (SAMRS) attempted in this work, but rejected based on various issues discovered through experimentation. The C* hydrogen bonding pattern implemented with the 5-methylzebularine heterocycle proved to be too weak a binder, as well as being problematic in chemical synthesis. The T* hydrogen bonding pattern implemented with the 3-methylpyrimidin-2-one heterocycle also proved to be inadequate as a binder.

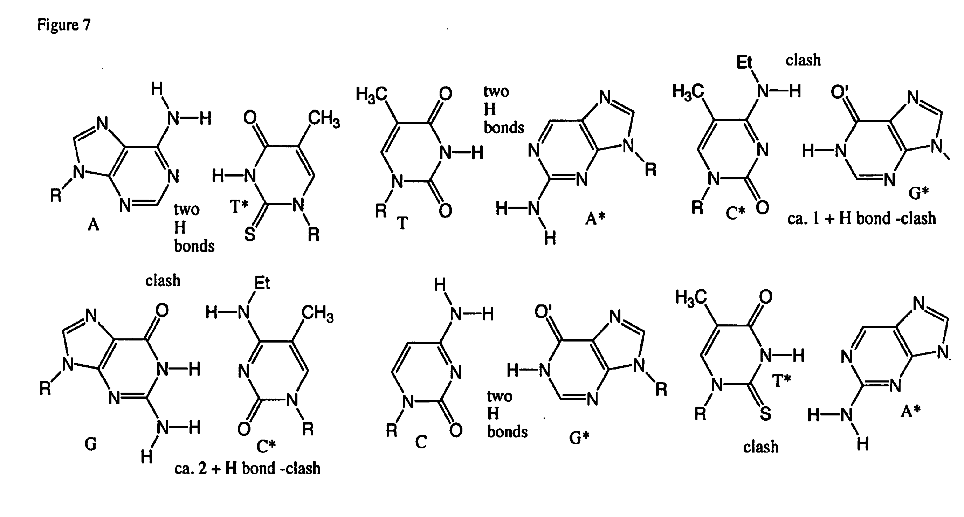

**Fig. 7****.** Self-Avoiding Molecular Recognition System (SAMRS) in their presently preferred implementation. A molecular recognition system that binds to complementary natural DNA, but not to complementary SAMRS sequences. The pairing of each of the complements of the SAMRS heterocycles (denoted by an asterisk *) with a standard nucleobase is joined by two hydrogen bonds, while pairs between any two size-complementary SAMRS components are joined by (at most) one hydrogen bond. Note that the G*-C* and A*-T* pairs in the wobble structure do not have two productive hydrogen bonds. In developing the SAMRS concept, rules for non standard nucleobase design determined in the Benner laboratory were exploited [Gey03]. Thus, none of the nucleobases pairs have uncompensated amino group in either of the grooves, having a negative charge, and are antiaromatic.

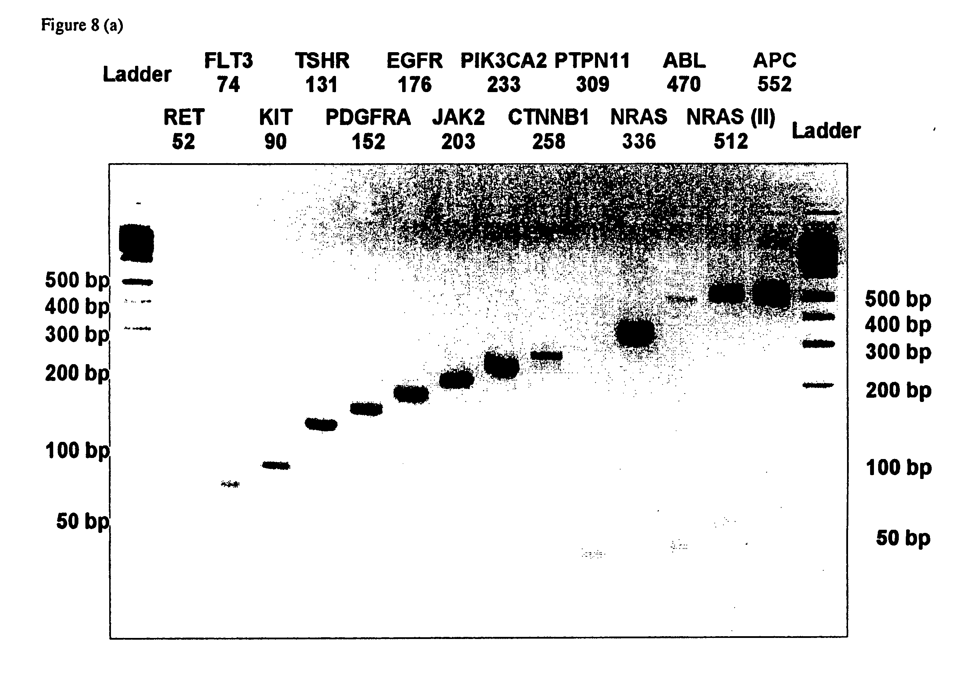

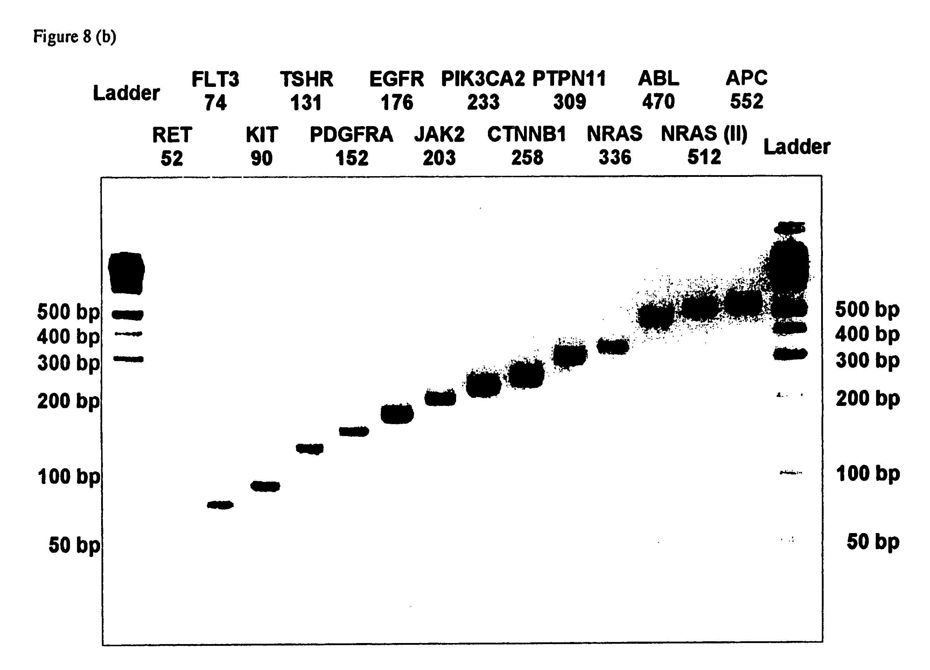

**Fig. 8****.** Monoplexed PCR experiments with standard primers (Fig. 8a) and SAMRS chimeric primers (Fig. 8b), with SAMRS hydrogen bonding patterns implemented as follows: T* implemented with 2-thiothymine; A* implemented with 2-aminopurine; G* implemented with hypoxanthine; C* implemented with N4-ethylcytosine, with primers targeted against various cancer genes of interest. Template: Human genomic DNA, 25 ng/25 microL Primers each 200 nM. dNTPs each 0.2 mM. Additional 5 mM MgCl₂ for SAMRS primers. Taq polymerase: 1.0 units/ 0.025 mL. 40 cycles: denature at 94 °C for 1 min, then annealing at 55 °C for SAMRS and 60 °C for standard primers for 1 min; then primer extension at 72 °C for 90 sec. Products were resolved on a 3% agarose gel and visualized by phosphorimager (one primer 5'-radiolabeled).

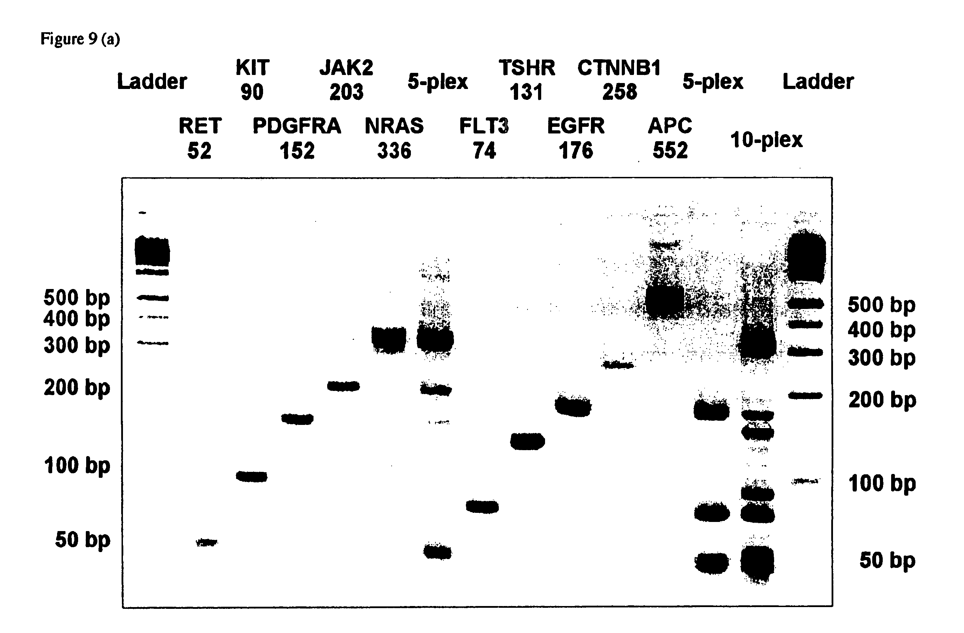

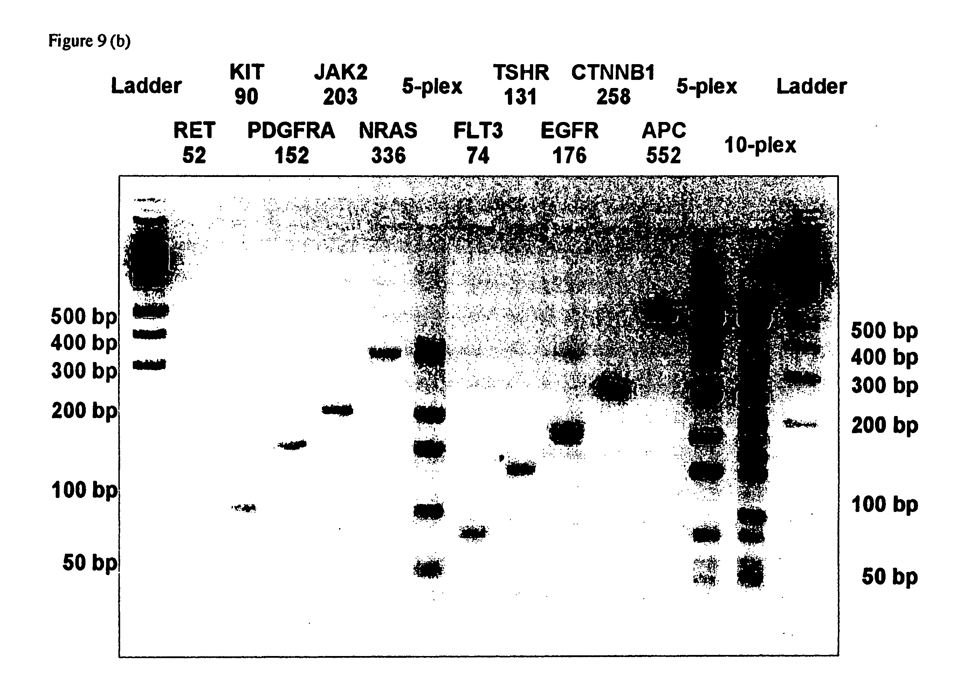

**Fig. 9****.** Five- and ten-fold multiplexed PCR experiments with standard primers (Fig. 9a) and SAMRS chimeric primers (Fig. 9b), as described in Example 18. SAMRS hydrogen bonding patterns implemented as follows: T* = 2-thiothymine; A* = 2-aminopurine; G* = hypoxanthine; C* = N4-ethylcytosine, with targets being various cancer genes. Template: Human genomic DNA, 25 ng/25 microL Primers each 200 nM. dNTPs each 1 mM.

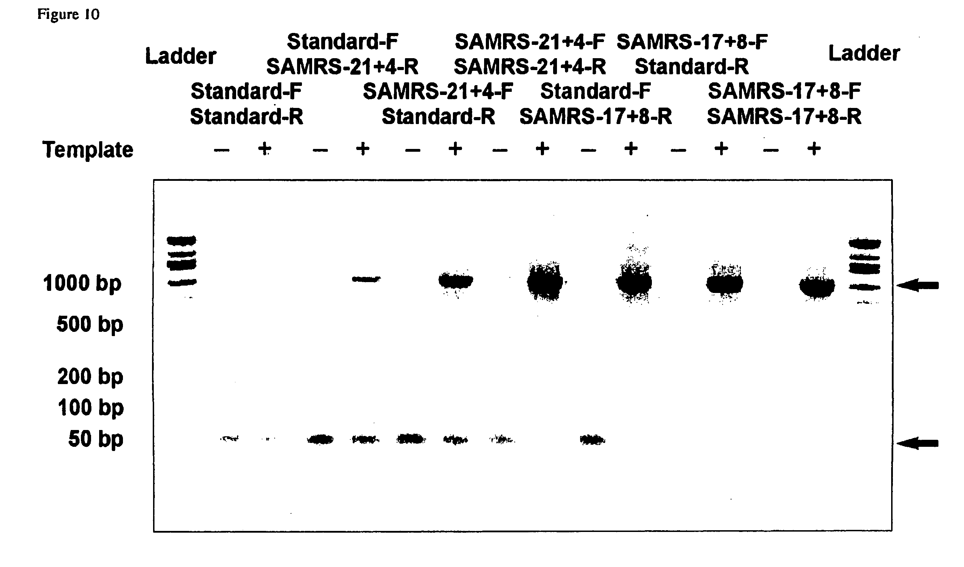

**Fig. 10****.** Amplification of the Taq polymerase gene with these primers at 500 nM concentration:
   SAMRS-21+4-F:5'-TAT CTG CGT GCC CTG TCT CTG* G*A*G* G-3' SEQ ID NO 1
   SAMRS-21+4-R:5'-CCA ATG CCA ACC TCT ACC TCC* A*G*A* G-3' SEQ ID NO 2
   SAMRS-17+8-F:5'-TAT CTG CGT GCC CTG TC*T* C*T*G* G*A*G* G-3' SEQ ID NO 3
   SAMRS-17+8-R:5'-CCA ATG CCA ACC TCT AC*C* T*C*C* A*G*A* G-3' SEQ ID NO 4
   An * following a letter indicates that the heterocycle is one of the presently preferred implementations of the indicated base. The primer pairs were deliberately designed to have overlap; with standard nucleotide components, no full length product ws generated, only primer dimer (lower arrow, 41 bps). DNA in 2% agarose gel was visualized with ethidium bromide.
**Fig. 11****.** Structures and abbreviations for various alternative implementations of C* examined.
**Fig. 12****.** Some primer extension of oligonucleotide templates containing scattered SAMRS components, reporting data collected in Example 13.
**Fig. 13**. Gel showing primer extensions using primers containing all SAMRS components reporting data collected in Example 14.
**Fig. 14****.** Gel showing polymerase read-through of templates containing consecutive SAMRS components, reporting data collected in Example 15.
**Fig. 15****.** Gel showing read through of thioT in templates, reporting data collected in Example 16.
**Fig. 16****.** Gel showing read through by DNA polymerase of SAMRS in templates at different concentrations of KCl, reporting data collected in Example 17.

### (i) Detailed Description of the Invention

Three issues require experimental examination in attempting to construct compositions of matter that comprise self-avoiding molecular recognition systems to support PCR reactions. First, in many PCR architectures, particularly those that are not "nested" [Bro97], the concentration of PCR primers must be very much higher than the concentration of the target that they are designed to amplify for the PCR reaction to be useful. Literature of the past decade (US Patent 5912340, [Lah08] and references therein) that describes the incorporation of diaminopurine, thiothymidine, ethylcytosine, inosine, and other SAMRS components into a single oligonucleotide to prevent its self-folding in secondary structure is not informative about this issue, as the SAMRS-containing oligonucleotide segments are present in exactly the same amounts as the segments of the same oligonucleotide that contain standard nucleotides. A PCR amplification requires preferably at least 100 fold amplification, and most preferably more than 100 fold amplification, and the total concentration of the components is preferably 100 nanomolar or greater. Experiments are needed to establish self-avoidance at these concentrations for any system proposed to do so, preferably by showing that formally matched primers do not form primer dimers at PCR-useful conditions. This is demonstrated for the instant system in Example 4.

Second, polymerases must be able to capture the primer-template complexes and extend them. Should this be demonstrated, then polymerases must be able to copy the extended product, the final elementary step in a PCR process. These too must be determined by experiment, as polymerases are notoriously idiosyncratic as to what unnatural species they will accept [Hor95]. Literature (such as US Patent 7371580) that concerns only binding properties does not provide experimental support. Further, literature that shows that non-standard SAMRS nucleoside triphosphates might be accepted as a substrate (see [Lah08] and references cited therein) does not inform about the ability of a primer containing non-standard nucleotides to be accepted by a polymerase, and certainly not whether a template containing non-standard nucleotides will be accepted by a polymerase. This demonstration found Example 4 (Fig. 10) for the preferred compositions, and Example 18 (Fig. 9) for multiplexed PCR.

This specification teaches that melting temperatures between a primer and a template are determinative as to whether the primer-template complex can be extended. This raises the third issue. While those experienced with natural DNA and its analogs have come to believe that certain generalizations (2'-OMe ribonucleotides contribute more to duplex stability than 2'-deoxyribonucleotides, RNA:DNA duplexes are more stable than DNA:DNA duplexes) apply to *all* heterocyclic systems that might support rule based molecular recognition in molecules resembling DNA. Example 3 shows that this is not the case for many of these rules for SAMRS nucleotides.

The nature of the invention taught here, why these issues needed to be resolved by experiment, and why the prior art did not teach the instant invention, are best understood through a description of the experiments that were performed to lead to the instant invention. Earlier generations of structures were explored in the development of this system before the preferred SAMRS components, the ones claimed here, were identified (Figures 4-6).

Initially, SAMRS nucleobase analogs (T*, A*, G* and C*) were designed to form exactly two hydrogen bonds to the complementary A, T, C, and G, respectively, but to have only one hydrogen bond joining the putative A*:T* pair and only one hydrogen bond joining the putative G*:C* pair. This was done (Fig. 5) by discarding the bottom hydrogen bonding group of G (an NH₂ group) to give G* (formally known as hypoxanthine), and discarding the top hydrogen bonding group (also an NH₂ group) from C to give C* (formally known as zebularine). Since natural adenine already lacks the bottom hydrogen bonding group (this would be an NH₂ group in 2,6-diaminopurine), we initially discarded the bottom hydrogen bonding group ofT (a C=O unit) to give T* (a pyridin-2-one C-glycoside) and the top hydrogen bonding group (an NH₂ group) of 2,6-diaminopurine to give A* (which is 2-aminopurine).

We expected that these T*:A, A*:T, C:*G, and G*:C nucleobase pairs would contribute to duplex stability to approximately the same extent as a natural A:T pair, which is also joined by two hydrogen bonds. Further, these designs were compatible with rules for designing non-standard nucleobases reported in [Gey03], where uncompensated carbonyl hydrogen bonding units were found experimentally to be compatible with duplex stability while uncompensated amino groups were not.

Routes [Lan00][Woo03][Sil99] were known to synthesize the 2'-deoxyriboside of pyrid-2-one, the first generation T*. But the nucleobase pair between 2-pyrid-2-one and adenine did not contribute adequately to duplex stability. Therefore, a methyl group was added to pyrid-2-one to exploit the well-known stabilizing effect of this group on duplex stability. This implementation of A* was facilitated by the commercial availability of the 2'-deoxyribonucleoside of 2-aminopurine as a protected phosphoramidite from Glen Research. The A* is the only heterocycle in the preferred structures that carries exocyclic functional groups that need protection for standard phosphoramidite synthesis. Alkaline conditions used to deprotect protected 2-aminopurine after its incorporation into an oligonucleotide may have been incompatible with zebularine, the first generation implementation of C*. Therefore, the phenoxyacetyl protecting group was used as the protecting group for the exocyclic amino group of 2-aminopurine.

The 2'-deoxyriboside of pyrimidin-2-one (the nucleobase found in zebularine, implementing the first generation C*) is also known [Viv04]. It was commercially available as the 5-methyl derivative from Glen Research [Sin01]. Inosine is commercially available in multiple forms, and can be synthesized inexpensively from the appropriate adenosine derivative through deamination. It may also be obtained as the 7-deazainosine analog.

Extensive experimentation with this first generation set of SAMRS components suggested that it needed improvement to provide useful compositions. The 5-methylpyrid-2-one:adenine pair and the 5-methylpyrimidin-2-one:guanine pair both contributed only poorly to duplex stability.

We therefore sought backbone modifications to increase the affinity of those primers. For example, it is standard practice to make the ribosides or the 2'-O-methylribosides to improve the stability of a base pair. This was tried here, and failed to provide the needed stabilization. Adding propynyl groups to position 5-of pyrimidines also used to increase the stability of base pairs. A series of oligonucleotides containing these were examined, but failed to achieve the desired level of performance of the self-avoiding oligonucleotides. Considering the possibility that zebularine forms a weak pair with G because it is a "push-push" electronic system, we prepared pyrimidin-2-ones having a 5-position methoxy group. This also did not give the needed stabilizing, even though some oligonucleotides containing various of these C* analogs did serve as primers and were adequate templates.

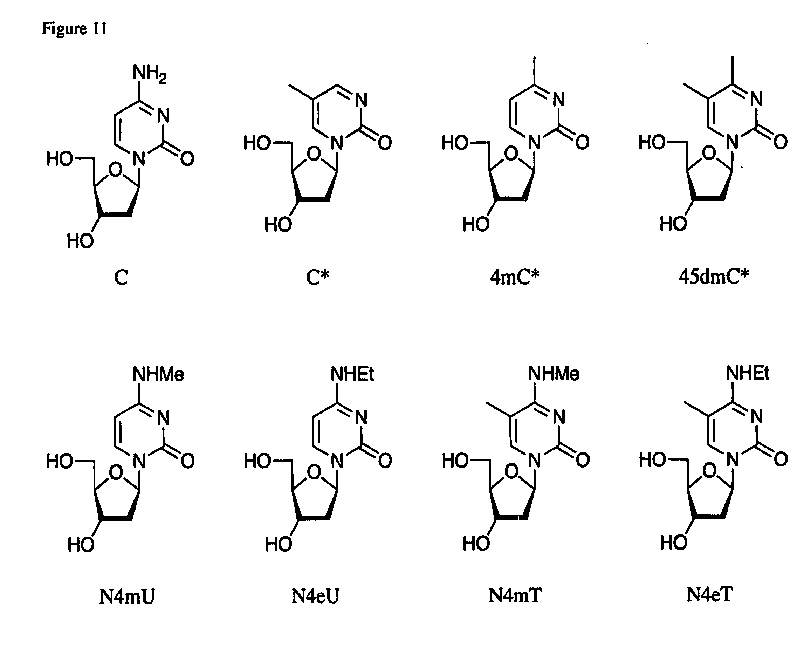

Various methylated derivatives of the zebularine heterocycle (Fig. 11) failed to perform to specification as well. Further, two types of chemical instability of zebularine heterocycles were found, including acid-catalyzed depyrimidinylation, and base-catalyzed Michael addition. These results were somewhat surprising, especially in light of literature that was appearing at the same time from laboratories attempting to incorporate non-standard nucleobases into oligonucleotides to prevent them from folding.

Adequate stabilization was obtained when the 5-methylpyrid-2-one implementation of T* was replaced by 2-thiothymidine. The sulfur of 2-thiothymidine is well known to be a poor hydrogen bond acceptor [Sis05], and therefore does not pair with the minor tautomer (for example) of isoguanine. 2-Thiothymidine may also be a better implementation of T* because it contributes more to duplex stability when paired with adenine than thymidine itself.

To replace a derivative of zebularine as the implementation of C*, we examined N⁴-methyl and N⁴-ethylcytosine. These have been incorporated into oligonucleotides as long ago as 1987 [Ono87] and are known to pair with G [Ngu98]. These provided the presently preferred implementations of the SAMRS disclosure (Figure 7). A series of melting temperatures were collected with these SAMRS nucleobases is provided throughout the examples. These provide a critical mass for generating heuristics that guide the practitioner in the art in using this system.

These rules are, as their name suggests, heuristics. As with standard DNA, standard RNA, and AEGIS molecular recognition systems, within a SAMRS system, predicting the binding properties of any sequence is subject to the same imprecision as predicting the properties of an arbitrary DNA or AEGIS molecule. Thus, as a general rule, if individuals of ordinary skill in the art wish to design a SAMRS sequence that binds to a preselected standard DNA molecule with a Tm of 25 °C, they would write down the preselected sequence in the 5'-to-3' direction, and then write below the SAMRS sequence in an antiparallel direction, matching a T* against every A in the preselected sequence, an A* against every T in the preselected sequence, a C* against every G in the preselected sequence, and a G* against every C in the preselected sequence.

We then turned to goal (b), finding conditions where SAMRS-containing oligonucleotides would serve as primers. Experiment found unexpectedly that duplexes held together entirely by base pairs joined by just two hydrogen bonds were less stable that expected by simple extrapolation from mixed sequence duplexes, and often needed to be much longer than mixed sequence duplexes. For example, oligonucleotides targeted to prime on some sequences were discovered by experiment to need to be as long as 25 nucleotides in length before they could efficiently be extended by DNA polymerases under standard PCR conditions.

This led to the discovery by experiment that primers worked best when they were composite. Self-avoiding behavior is, of course, most desired at the 3'-end of a primer in mixtures containing a plurality of primers, as complementarity here without SAMRS components allows the primer fragments to overlap, and these overlaps, upon incubation with triphosphates and a polymerase, leads under PCR conditions, to primer dimers. Conversely, the 5'-end of a primer need not be SAMRS to avoid primer dimer formation.

The instant invention is therefore based on several discoveries about the interaction between polymerases and SAMRS oligonucleotides, including:
(a) Polymerases are able to accept as primers oligonucleotides containing only SAMRS nucleobases (including cases where the 3-end is not a SAMRS nucleobase), but only if the oligonucleotide is at least 15 nucleotides long, and preferably longer than 15 nucleotides.
(b) DNA polymerases are able to use in PCR architectures primers containing SAMRS nucleobases, including N⁴-ethylcytosine, where we had expected the side chain to be rejected in a template, and inosine, where we had expected thermostable polymerases to reject it as a deamination product of adenosine. In contrast, PCR amplification was not supported by earlier generation SAMRS components.
(c) Self-avoiding behavior is demonstrated, even at concentrations of primers greater than 100 nM. Further, self-avoidance is possible in formally matched primer pairs as long as the 3'-ends contain SAMRS components, preferably four but more preferably up to eight, multiplexed PCR can be obtained with as many as 30 SAMRS primers in a library, amplifying 15 amplicons in one pot, without any effort made to optimize the primers to avoid inter-primer 3'-end complementarity, as is normally done in multiplexed PCR of this dimension.
(d) The self-avoiding utility arising from placing SAMRS components at the 3'-ends of members of a mixture of primers is not lost if the last 3'-terminal nucleotide carries a standard nucleobase, even if the primers are fully formally complementary (Example 4, Fig. 10). This expedient that lowers the cost of the primers by allowing primers to be synthesized on standard controlled pore glass supports that are commercially available.
(e) A critical mass of melting temperature data are presented to support heuristic rules.
(f) Some heuristic rules can be transferred from DNA to SAMRS systems, while others cannot. Thus, longer SAMRS oligonucleotides have higher Tₘ values than shorter, where increasing the length from 18 to 25mer in Example 7 increased the Tₘ from 30.8 to 42.0 °C. Likewise, increasing the concentration of salt (in Example 7) increases the Tₘ. Likewise, replacing 2-aminopurine as an implementation of the A* by 2,6-diaminopurine increases Tₘ (Example 8). Increasing the concentration of KCl decreased pausing by polymerases as they encountered SAMRS in a template (Example 17). However, the general heuristic rule that replacing a 2'-deoxyribose as the supporting sugar by a 2'-O-methylribose does not work well with SAMRS nucleobases, and the Tₘ scales severely downwards as all of the standard:standard base pairs are replaced with SAMRS:standard pairs.
(g) Further issues relate directly to the choice of a polymerase in the use of SAMRS components in PCR. For example, the preferred compound for a G* analog was inosine. However, inosine is a deamination product of adenosine, and many thermostable polymerases from organisms that live at very high temperatures are known to pause at inosine, presumably to permit the repair of this common defect. Hence, Taq is the preferred polymerase for the preferred SAMRS components.

Further, it is demonstrated that SAMRS-containing oligonucleotides can be prepared with a 5'-phosphate label, and where the 5'-OH group is replaced by a 5'-amino group (and is therefore useful in the architecture shown in Figure 2. Further modification at the 5'-end, to include a biotin, a capture tag, or a fluorescent tag, are also taught herein.

Last, for PCR primer pairs, it is taught that these are designed to contain SAMRS components where the first primer in the pair is complementary to a target sequence at a preselected portion, and therefore binds to this portion of the target sequence, and the second member in the pair is formally the same as a segment of the same target sequence at a position upstream (that is in the 5'-direction) from this portion, where "formally the same" means that A or A* in the second primer corresponds to an A in the target sequence, T or T* in the second primer corresponds to a T in the target sequence, G or G* in the second primer corresponds to a G in the target sequence, and C or C* in the second primer corresponds to a C in the target sequence. This simply means that the second primer is complementary to the product obtained by extending the first primer using the target as the template, meaning that the second primer will prime on the first product. These are the relationships between the binding site of any pair of primers that is useful in PCR.

The length of the amplicon (excluding the portion derived from the primers themselves) is determined by this spacing. Useful amplicons are of length 10-1000, preferably, more preferably from 10 to 400.Thus, nucleotide on the target that binds the 3'-end of the first primer should be 10 nucleotides in the 3'-direction from the nucleotide in the target that is formally equivalent to the 3'-end nucleotide of the second primer.

### Examples

### Example 1. Preparation of the base-unprotected thiothymidine phosphoramidite. 5'-O-(4,4'-dimethoxytrityl)-2'-deoxy-2-thiothymidine.

To a solution of 2-thiothymidine (Berry & Associates, 1.95 g, 7.55 mmol) in anhydrous pyridine (50 mL) was added DMTrCl (2.94 g, 8.68 mmol). The mixture was stirred at room temperature for 20 h. The reaction was then quenched by addition of MeOH (10 mL) and the solvents were removed by evaporation. The residue was dissolved in AcOEt, washed with distilled water and brine, dried with anhydrous Na₂SO₄ and evaporated. The residue was purified on silica gel column chromatography using 67% hexane in AcOEt as the eluent to give 3.90 g of the 5'-dimethoxytritylated species (92%) as white foam.

NMR (Varian Mercury 300 MHz spectrometer): ¹H-NMR (CDCl₃, 300 MHz): δ 1.45 (s, 3H); 2.25-2.34 (m, 1H); 2.59-2.67 (m, 1H); 3.36-3.41 (dd, 1H); 3.53-3.57 (dd, 1H); 3.77 (s, 6H); 4.11 (m, 1H); 4.60 (m, 1H); 6.86 (t, 1H); 6.81-7.40 (m, 13H); 7.84 (s, 1H). ¹³C-NMR (CDCl₃): δ 12.3, 41.3, 55.5, 63.2, 71.9, 86.8, 87.2, 90.1, 113.6, 116.7, 127.5, 128.3, 128.3, 130.3, 135.5, 136.9, 144.5, 159.0, 161.1, 174.3. ESI-TOF (+) MASS: *m*/*z* [M + Na]⁺ calcd for C₃₁H₃₂N₂O₆S + Na: 583.1873; found: 583.1897.

### 5'-{O-[(4,4'-dimethoxytrityl)-2'-deoxy-2-thiothymidine]}-3'-[2-cyanoethyl bis(1-methylethyl)phosphoramidite.

To a solution of the 5'-dimethoxytritylated species from above (300 mg, 0.54 mmol) in anhydrous CH₂Cl₂ (5 mL) was added *N,N*-diisopropylethylamine (235 µL, 1.35 mmol) followed by 2-cyanoethyl-*N,N-*diisopropylchlorophosphoramidite (181 µL, 0.81 mmol). The mixture was stirred at room temperature for 2 h. To the mixture was added AcOEt, washed with distilled water and brine, dried with Na₂SO₄ and evaporated. The residue was purified on neutral silica gel column chromatography using 33% hexane in AcOEt as the eluent to give 348 mg of 3 (85%) as white foam. NMR (Varian Mercury 300 MHz spectrometer): ¹H-NMR (CDCl₃, 300 MHz): δ 1.04-1.18 (m, 12H); 1.40 and 1.42 (each s, 3H); 2.26-2.36 (m, 1H); 2.42 and 2.63 (each t, 2H); 2.62-2.79 (m, 1H); 3.31-3.88 (m, 6H); 3.80 (s, 6H); 4.18 (m, 1H); 4.67 (m, 1H); 6.92 (m, 1H); 6.82-7.42 (m, 13H); 7.88 and 7.92 (each s, 1H); 9.36 (br s, 1H). ³¹P-NMR (CDCl₃, 121 MHz): δ (ppm, rel to external standard H₃PO₄ = 0) = 149.7; 150.4. ESI-TOF (+) MASS: *m*/*z* [M + Na]⁺ calcd for C₄₀H₄₉N₄0₇PS + Na: 783.2952; found: 783.2909.

High Performance Liquid Chromatography. HPLC purification of the oligonucleotides is accomplished as described below. Analytical HPLC is also used for purification of the oligonucleotides. Analytical HPLC; Waters 600S Controller, Waters™ 616 Pump, Waters™ 486 Tunable Absorbance Detector; Preparative HPLC; Waters PrepLC System Controller, Waters PrepLC 4000 System, Waters 486 Tunable Absorbance Detector; Reverse phase column; Waters Nova-Pak C 18 (3.9 x 150 mm) for analytical HPLC, Waters Nova-Pak HR C18 (7.8 x 300 mm) for preparative HPLC; Ion-exchange column; DIONEX DNAPac PA-100 (4 x 250 mm) for analytical HPLC, DIONEX DNAPac PA-100 (9 x 250 mm) for preparative HPLC. Reverse phase; A buffer = 25 mM TEAA (or 25 mM NH₄OAc), B buffer = 20% MeCN in 25 mM TEAA (or 25 mM NH₄OAc). Ion exchange; 25 mM TEAA, 200 mM NaCl, B solution = 25 mM TEAA, 1 M NaCl.

### Example 2. Preparation of presently preferred SAMRS oligonucleotides

Phosphoramidite chemistry has made routine the synthesis of the 4*ⁿ* of DNA and RNA molecules having n nucleotides in a sequence. Therefore, it is not necessary to have in possession every one of those sequences to enable the practice of an invention that claims all of those sequences. Analogously, the compositions of the instant invention are prepared by phosphoramidite synthesis, where the outcome of the synthesis is not dependent on the precise order in which nucleoside phosphoramidites are added. For several of the SAMRS components, phosphoramidites are commercially available. Literature procedures are available to make others [Ono87][Ngu98]Oligonucleotides containing the presently preferred SAMRS components were prepared as follows:
2'-Deoxy-5'-dimethoxytritylinosine-3'-O-(3-cyanoethyl-diisopropylaminophosphoramidite) was purchased from Glen Research and dissolved in anhydrous acetonitrile to a final concentration of 0.12 M. 2'-Deoxy-5'-dimethoxytrityl-2-thiothymidine-3'-O-(3-cyanoethyl-diisopropylaminophosphoramidite), prepared as described above, was dissolved in anhydrous acetonitrile (final concentration 0.12 M). 2'-Deoxy-5'-dimethoxytrityl-N⁴-ethylcytidine-3'-O-(3-cyanoethyl-diisopropylaminophosphoramidite) is available from Glen Research (the material used in this work was synthesized from thymidine) and also dissolved in anhydrous acetonitrile (final concentration 0.12 M). Finally, 2'-deoxy-5'-dimethoxytrityl-2-aminopurine-3'-O-(3-cyanoethyl-diisopropylaminophosphoramidite) protected as its N-phenoxyacetyl derivative (prepared from 2'-deoxyriboside of 2-amino purine, from Berry and Associates) was dissolved in anhydrous acetonitrile (final concentration 0.12 M). Bottles containing these were installed on an Applied Biosystems 394 DNA synthesizer (Foster City, CA), and the synthesis was initiated on a standard controlled pore glass support with the standard nucleotide attached, as desired for the 3'-end. Coupling times were 10 min. A solution of 3% dichloroacetic acid in dichloromethane was used for 5'-detritylation. Following completion of the synthesis, the products were released by treatment with concentrated NH₄OH at room temperature for 16 h. The solutions were then frozen and lyophilized. The oligonucleotides were purified on 20 % PAGE containing 7 M urea.

As a comment on the synthesis, it was found that the nitrogen of N⁴-ethylcytidine did not need protection. Further, it was found that harsher deprotection conditions led to byproducts suggestive of a substantial loss of the sulfur from 2-thioT, a problem documented in literature.

For this reason, phenoxyacetyl was used to protect 2-aminoadenine. The dimethylformamidine protected phosphoramidite of 2-aminopurine-5'-dimethoxytrityl-3'-deoxynucleoside should also work, and it is at this time commercially available from Glen Research.

### Example 3. Melting temperatures of oligonucleotides containing various SAMRS components, preferred and not preferred.

### Example 3(a)

To explore the melting temperatures of SAMRS systems where 5-methylzebularine implemented a C* hydrogen bonding pattern, pairs of oligonucleotides were synthesized with a single * analog at the positions indicated by X and Y. Melting temperature measurements of the duplexes are done at 260 nm with 1 mL samples at a concentration of 3 µM per single strand.

**Table 3.1. Sequences used in these melting temperature studies**

| |
|---|
| SAMRS-Tm1: 5'-ACCAAGCXATCAAGT-3' X = A, SEQ ID NO 5 |
| SAMRS-Tm1: 5'-ACCAAGCXATCAAGT-3' X = 2-aminopurine, SEQ ID NO 6 |
| SAMRS-Tm1: 5'-ACCAAGCXATCAAGT-3' X = T, SEQ ID NO 7 |
| SAMRS-Tm1: 5'-ACCAAGCXATCAAGT-3' X = 2-thiothymine, SEQ ID NO 8 |
| SAMRS-Tm1: 5'-ACCAAGCXATCAAGT-3' X = G, SEQ ID NO 9 |
| SAMRS-Tm1: 5'-ACCAAGCXATCAAGT-3' X = hypoxanthine, SEQ ID NO 10 |
| SAMRS-Tm1: 5'-ACCAAGCXATCAAGT-3' X = C, SEQ ID NO 11 |
| SAMRS-Tm1: 5'-ACCAAGCXATCAAGT-3' X = 5-methylpyrimidin-2-one, SEQ ID NO 12 |
| SAMRS-Tm1-C: 3'-TGGTTCGYTAGTTCA-5' Y = A, SEQ ID NO 13 |
| SAMRS-Tm1-C: 3'-TGGTTCGYTAGTTCA-5' Y = 2-aminopurine, SEQ ID NO 14 |
| SAMRS-Tm1-C: 3'-TGGTTCGYTAGTTCA-5' Y = T, SEQ ID NO 15 |
| SAMRS-Tm1-C: 3'-TGGTTCGYTAGTTCA-5' Y = 2-thiothymine, SEQ ID NO 16 |
| SAMRS-Tm1-C: 3'-TGGTTCGYTAGTTCA-5' Y = G, SEQ ID NO 17 |
| SAMRS-Tm1-C: 3'-TGGTTCGYTAGTTCA-5' Y = hypoxanthine, SEQ ID NO 18 |
| SAMRS-Tm1-C: 3'-TGGTTCGYTAGTTCA-5' Y = C, SEQ ID NO 19 |
| SAMRS-Tm1-C: 3'-TGGTTCGYTAGTTCA-5' Y = 5-methylpyrimidin-2-one, SEQ ID NO 20 |

These substituted heterocycles implemented the SAMRS binding patterns: A* = 2-aminopurine; T* = 2-thiothymine; C* = 5-methylpyrimidin-2-one; G* = hypoxanthine.

**Table 3.2. Experimentally determined melting temperatures**

| X\Y | T | T* | A | A* | C | C* | G | G* |
|---|---|---|---|---|---|---|---|---|
| A | 55.5 | 56.8 | 43.7 | 46.5 | 45.1 | 43.5 | 46.7 | 49.8 |
| A* | 54.5 | 52.0 | 46.8 | 45.5 | 48.1 | 44.0 | 45.8 | 46.8 |
| T | 46.3 | 48.0 | 54.0 | 52.5 | 44.6 | 45.0 | 48.4 | 46.3 |
| T* | 47.0 | 50.0 | 54.0 | 50.3 | 40.9 | 41.3 | 44.6 | 45.1 |
| G | 49.5 | 47.0 | 47.0 | 45.1 | 58.8 | 52.0 | 47.0 | 46.0 |
| G* | 48.8 | 47.0 | 50.5 | 45.1 | 54.1 | 49.3 | 46.0 | 46.3 |
| C | 44.0 | 40.6 | 42.8 | 47.1 | 43.8 | 41.0 | 59.0 | 52.6 |
| C* | 44.0 | 42.0 | 42.0 | 43.0 | 41.1 | 39.5 | 52.0 | 47.8 |

Tₘ data in Table 3.2 show that in these particular implementations, the A*:T and T*:A pairs contributed to duplex stability to an extent similar to the contribution made by an A:T pair; the T*:A pair is known in the literature to contribute slightly more. This is confirmed by four melting temperature experiments that capture these pairs in different contexts. The A*:T* pair, in contrast, contributed less. This system is presently not preferred based on these empirical data that show that the 5-methylpyrimidin-2-one:G pair does not contribute to duplex stability substantially more than the A*:T* pair. Interestingly, these experimental results contradict proposals in the art by Gamper *et al.* [Gam06] that methylpyrimidin-2-one derivatives might serve as an implementation of C* (although this proposal concerned creating nucleic acids that do not fold on themselves, not nucleic acids that might serve as primers.

### Example 3(b)

One expedient standard in the literature concerning non-SAMRS molecular recognition systems seeks to improve the stability of duplexes by presenting the heterocycles on 2'-OMe sugars rather 2'-deoxyribose sugars. Experiments were required to determine whether this worked in the SAMRS system. Here, pairs of oligonucleotides were synthesized with a single SAMRS analog at positions indicated by X and Y. Melting temperature were measured at 260 nm with 1 mL samples at 3 µM per single strand.

**Table 33. Sequences used in these melting temperature studies**

| |
|---|
| SAMRS-Tm1: 5'-ACCAAGCXATCAAGT-3' X = A, SEQ ID NO 5 |
| SAMRS-Tm1: 5'-ACCAAGCXATCAAGT-3' X = 2-aminopurine, SEQ ID NO 6 |
| SAMRS-Tm1: 5'-ACCAAGCXATCAAGT-3' X = T, SEQ ID NO 7 |
| SAMRS-Tm1: 5'-ACCAAGCXATCAAGT-3' X = 2-thiothymine, SEQ ID NO 8 |
| SAMRS-Tm1: 5'-ACCAAGCXATCAAGT-3' X = G, SEQ ID NO 9 |
| SAMRS-Tm1: 5'-ACCAAGCXATCAAGT-3' X = hypoxanthine, SEQ ID NO 10 |
| SAMRS-Tm1: 5'-ACCAAGCXATCAAGT-3' X = C, SEQ ID NO 11 |
| SAMRS-Tm1: 5'-ACCAAGCXATCAAGT-3' X = 5-methyl-pyrimidin-2-one, SEQ ID NO 12 |
| SAMRS-Tm1-C: 3'-TGGTTCGYTAGTTCA-5' Y = A, SEQ ID NO 13 |
| SAMRS-Tm1-C: 3'-TGGTTCGYTAGTTCA-5' Y = 2-aminopurine, SEQ ID NO 14 |
| SAMRS-Tm1-C: 3'-TGGTTCGYTAGTTCA-5' Y = T, SEQ ID NO 15 |
| SAMRS-Tm1-C: 3'-TGGTTCGYTAGTTCA-5' Y = 2-thiothymine, SEQ ID NO 16 |
| SAMRS-Tm1-C: 3'-TGGTTCGYTAGTTCA-5' Y = G, SEQ ID NO 17 |
| SAMRS-Tm1-C: 3'-TGGTTCGYTAGTTCA-5' Y = C, SEQ ID NO 19 |
| SAMRS-Tm1-C: 3'-TGGTTCGYTAGTTCA-5' Y = hypoxanthine, SEQ ID NO 21 |
| SAMRS-Tm1-C: 3'-TGGTTCGYTAGTTCA-5' Y = 5-methylpyrimidin-2-one SEQ ID NO 22 |

The following heterocycles implemented the SAMRS strategy: A* = 2-aminopurine; T* = 2-thiothymine; C* = 5-methyl-pyrimidin-2-one; G* = hypoxanthine. Interestingly, experimental work showed that the "2'-OMe generalization" that applies to certain other DNA-like molecular recognition systems does not apply here. The C*:G and G*:C pairs supported on a 2'-deoxyribose sugar have Tₘs of 52.0/52.0 and 54.1/52.6 (respectively, in two contexts). The Tₘs are all lower when one of the SAMRS implementations is supported on a 2-'OMe sugar.

**Table 3.4. Melting temperatures with 2'-OMe nucleotides**

| X:Y | T | T* | A | A* | C | OMeC* | G | OMeG* |
|---|---|---|---|---|---|---|---|---|
| A | 55.5 | 56.8 | 43.7 | 46.5 | 45.1 | 43.0 | 46.7 | 48.3 |
| A* | 54.5 | 52.0 | 46.8 | 45.5 | 48.1 | 44.1 | 45.8 | 46.0 |
| T | 46.3 | 48.0 | 54.0 | 52.5 | 44.6 | 43.0 | 48.4 | 46.0 |
| T* | 47.0 | 50.0 | 54.0 | 50.3 | 40.9 | 42.3 | 44.6 | 45.3 |
| G | 49.5 | 47.0 | 47.0 | 45.1 | 58.8 | 51.0 | 47.0 | 44.8 |
| OMeG* | 48.0 | 47.0 | 49.0 | 46.0 | 52.8 | 49.0 | 45.1 | 44.8 |
| C | 44.0 | 40.6 | 42.8 | 47.1 | 43.8 | 42.0 | 59.0 | 51.0 |
| OMeC* | 42.8 | 42.1 | 42.0 | 43.0 | 42.8 | 40.0 | 51.5 | 49.0 |

### Example 3(c)

Another often cited generalization, tested to date only in non-SAMRS systems, is that RNA:DNA duplexes are more stable than the corresponding DNA:DNA duplexes with analogous sequences. To explore the melting temperatures of SAMRS systems where one of the strands was RNA and the other was DNA, pairs of oligonucleotides were synthesized with a single * analog at the positions indicated by X and Y. Melting temperature measurements of duplexes were done at 260 nm with 1 mL samples at a concentration of 3 µM per single strand.

**Table 3.5. Sequences used in these melting temperature studies**

| |
|---|
| SAMRS-Tm1: 5'-r(ACCAAGCXAUCAAGU)-3' Y = A, SEQ ID NO 23 |
| SAMRS-Tm1: 5'-r(ACCAAGCXAUCAAGU)-3' Y = 2-aminopurine, SEQ ID NO 24 |
| SAMRS-Tm1: 5'-r(ACCAAGCXAUCAAGU)-3' Y = T, SEQ ID NO 25 |
| SAMRS-Tm1: 5'-r(ACCAAGCXAUCAAGU)-3' Y = 2-thiothymine,SEQ ID NO 26 |
| SAMRS-Tm1: 5'-r(ACCAAGCXAUCAAGU)-3' Y = G, SEQ ID NO 27 |
| SAMRS-Tm1: 5'-r(ACCAAGCXAUCAAGU)-3' Y = C, SEQ ID NO 28 |
| SAMRS-Tm1: 5'-r(ACCAAGCXAUCAAGU)-3' Y = hypoxanthine, SEQ ID NO 29 |
| SAMRS-Tm1: 5'-r(ACCAAGCXAUCAAGU)-3' Y = 5-methylpyrimidin-2-one, SEQ ID NO 30 |
| SAMRS-Tm1-C: 3'-TGGTTCGYTAGTTCA-5' Y = A, SEQ ID NO 13 |
| SAMRS-Tm1-C: 3'-TGGTTCGYTAGTTCA-5' Y = 2-aminopurine, SEQ ID NO 14 |
| SAMRS-Tm1-C: 3'-TGGTTCGYTAGTTCA-5' Y = T, SEQ ID NO 15 |
| SAMRS-Tm1-C: 3'-TGGTTCGYTAGTTCA-5' Y = 2-thiothymine, SEQ ID NO 16 |
| SAMRS-Tm1-C: 3'-TGGTTCGYTAGTTCA-5' Y = G, SEQ ID NO 17 |
| SAMRS-Tm1-C: 3'-TGGTTCGYTAGTTCA-5' Y = hypoxanthine, SEQ ID NO 18 |
| SAMRS-Tm1-C: 3'-TGGTTCGYTAGTTCA-5' Y = C, SEQ ID NO 19 |
| SAMRS-Tm1-C: 3'-TGGTTCGYTAGTTCA-5' Y = 5-methylpyrimidin-2-one, SEQ ID NO 20 |

**Table 3.6**

| X:Y | T | T* | A | A* | C | C* | G | G* |
|---|---|---|---|---|---|---|---|---|
| A | 43.8 | 45.3 | 31.5 | 32.5 | 34.0 | 34.0 | 32.3 | 35.0 |
| A* | | 52.0 | | 45.5 | | 44.0 | | 46.8 |
| U | 34.3 | 37.0 | 41.0 | 41.3 | 33.0 | 33.0 | 35.3 | 33.8 |
| T* | | 50.0 | | 50.3 | | 41.3 | | 45.1 |
| G | 41.3 | 36.5 | 33.0 | 33.5 | 48.3 | 43.3 | 34.8 | 35.0 |
| G* | | 47.0 | | 45.1 | | 49.3 | | 46.3 |
| C | 29.0 | 29.3 | 28.3 | 33.0 | 28.8 | 31.0 | 42.8 | 35.8 |
| C* | | 42.0 | | 43.0 | | 39.5 | | 47.8 |

The following implementations were again: A* = 2-aminopurine; T* = 2-thiothymine; C* = 5-methylpyrimidin-2-one, G* = hypoxanthine. Again, experimental results with the SAMRS system gave results that contradicted the rule, commonly cited for other heterocycles, that RNA:DNA duplexes are more stable than DNA:DNA duplexes. Indeed, some of the duplexes appeared to be less stable than would be useful, at least for oligonucleotides having this length.

### Example 4. Melting, primer extension, and PCR with preferred SAMRS components

With these results, we turned to alternative implementations of C*. Experiments done to develop these are presented given below. The preferred implementation based on melting temperatures for C* was N⁴-ethylcytosine. Experiments were required to determine if this non-standard nucleotide, as well as others in the presently preferred set, would be accepted by polymerases as primers and, more important for PCR, as templates. Chimeric primers designed to create a 1100 bp PCR amplicon from the Taq gene. These primers had a 5'-segment built from standard nucleotides and the 3'-end built from SAMRS nucleotides (with the last 3'-nucleotide being standard to allow a lower cost of synthesis), with A* =2-aminopurine; T* =2-thiothymine; C* =N⁴-ethylcytosine; G* =hypoxanthine Their melting temperatures were determined by UV (Cary; 20 mM Na cacodylate, pH 7.0; 100 mM NaCl, 3 microM each oligonucleotide).

**Table 4.1 Tₘ values for primers used to amplify the gene for Taq DNA polymerase**

| sequence | Tₘ (°C) |
|---|---|
| 5'-TATCTGCGTGCCCTGTCTCTGGAGG-3' SEQ ID NO 31 | 74.0 |
| 3'-ATAGACGCACGGGACAGAGACCTCC-5' SEQ ID NO 32 | |
| 5'-TATCTGCGTGCCCTGTCTCTG*G*A*G*G -3' SEQ ID NO 1 | 68.8 |
| 3'-ATAGACGCACGGGACAGAGAC C T C C -5' SEQ ID NO 32 | |
| 5'-TATCTGCGTGCCCTGTC*T*C*T*G*G*A*G*G -3' SEQ ID NO 3 | 65.0 |
| 3'-ATAGACGCACGGGACAG A G A C C T C C -5' SEQ ID NO 32 | |

The PCR experiment was performed with template at 66 pM, the primers were at 500 nM, triphosphates at 0.2 mM, Mg⁺⁺ at 2.5 mM, with 1 unit of Taq polymerase. Cycles (25) were run at 94 °C (1 min), 55 °C (1 min), then 72 °C (2 min). Results are shown in Fig. 10. Note that the primers were deliberately designed to produce primer dimers when implemented using standard nucleotides. Accordingly, no amplicon is observed with these primers, unless they have SAMRS components at their 3'-end. Adding SAMRS components to the 3'-end reduces, and then eliminates primer-dimer, and increases the amount of full-length amplicon. This represents the first experimental demonstration of self-avoiding properties in primer extension experiments, and is therefore a key discovery that supports the patentability of the instant invention.

These results also show that in this implementation, SAMRS can support PCR.

The concentration of primers is important, as it quantitates self-avoidance. Primers at high dilution, of course, will always avoid each other. However, to be useful in PCR, primers must be present at high concentration and at high stoichiometric ratio over target; their concentration, in fact, determines the fold amplification. Hence, these concentrations are appropriate parts of aspects in the instant disclosure.

Several comments are relevant about experiments that failed, most of which are not shown. First, 25mers built entirely from SAMRS components (except for the 3'-terminal nucleotide) primed considerably less well. Further, they did not support PCR amplification to a detectable (or useful) extent. Second, polymerases from extreme thermophiles did not perform PCR, even with this preferred implementation. Without being bound by theory, this may be because hypoxanthine, being a deamination product of adenine that may arise in excessive amounts at very high temperatures, may be rejected by polymerases that evolved to work at very high temperatures. Third, as the basis for the presently preferred primer sequences, it was found that SAMRS components at the 3'-end at from four to eight sites were sufficient to render these self-avoiding at the high concentrations of primers used. Last, having a standard nucleotide at the 3'-position (unexpectedly) did not destroy the self-avoiding property that makes pairs of SAMRS-containing primers useful for PCR, and multiplexed PCR in particular.

### Example 5. Primers targeted against human retinoblastoma RNA or cDNA (RET).

Considerable work was then done to develop a critical mass of melting temperature data for primers that might be used in PCR, where the primers have from 4 to 8 SAMRS components in their 3'-ends (but not necessarily in the very 3'-terminal end. While these data show the expected decrease in Tₘ as pairs joined by three hydrogen bonds are replaced by pairs joined by just two, they showed unexpectedly that a duplex joined nearly entirely by base pairs joined by just two (as is the case when the pair is made nearly entirely from SAMRS:standard pairs) was extremely unstable. Without being bound by theory, this result, not obvious from the prior art, is possibly an explanation for the failure of fully SAMRS-labeled primers to support PCR.

**Table 5.1. Tₘ values for primers useful for amplifying human retinoblastoma cDNA**

| sequence | Tₘ (°C) |
|---|---|
| 5'-C*C*A*G*G*A*T*C*C*A*C*T*G*T*G*C*G*A*C*G*A*G*C*T*G-3' SEQ ID NO 33 | 40.3 |
| 3'-G G T C C T A G G T G A C A C G C T G C T C G A C-5' SEQ ID NO 34 | |
| 5'-C G C A C G G T G A T C G C A G C C G C T G T C C-3' SEQ ID NO 35 | 44.0 |
| 3'-G C*G*T*G*C*C*A*C*T*A*G*C*G*T*C*G*G*C*G*A*C*A*G*G*-5' SEQ ID NO 36 | |
| 5'-CCA*GGA*T*CCA*CT*GT*GCGA*CGA*GCT*G -3' SEQ ID NO 37 3'-GGT CCT A GGT GA CA CGCT GCT CGA C -5' SEQ ID NO 34 | 74.8 |
| 5'-CGCA CGGT GA T CGCA GCCGCT GT CC-3' SEQ ID NO 35 3'-GCGT*GCCA*CT*A*GCGT*CGGCGA*CA*GG-5' SEQ ID NO 38 | 80.3 |
| 5'-CCAGGATCCACTGTGCG*A*C*G*A*G*C*T*G -3' SEQ ID NO 39 | 62.3 |
| 3'-GGTCCTAGGTGACACGC T G C T C G A C -5' SEQ ID NO 34 | |
| 5'-CG C A C G G T G ATCGCAGCCGCTGTCC-3' SEQ ID NO 35 | 70.5 |
| 3'-GC*G*T*G*C*C*A*C*TAGCGTCGGCGACAGG-5' SEQ ID NO 40 | |
| 5'-CCAGGATCCACTGTGCGACGA*G*C*T*G -3' SEQ ID NO 41 | 72.0 |
| 3'-GGTCCTAGGTGACACGCTGCT C G A C -5' SEQ ID NO 34 | |
| 5'-CG C A C GGTGATCGCAGCCGCTGTCC-3' SEQ ID NO 35 | 75.7 |
| 3'-GC*G*T*G*CCACTAGCGTCGGCGACAGG-5' SEQ ID NO 42 | |

### Example 6. Melting temperatures with primers targeted against Taq and the human retinoblastoma transcript (RET) as a function of length.

To further develop a critical mass of Tₘ data from which those skilled in the art and taught by this disclosure to use SAMRS components effectively, the Tₘs of all-SAMRS oligonucleotides (except for the very last nucleotide at the 3'-end, which is standard) were compared with the chimeric species having the same sequence. Conditions: 20 mM Na cacodylate (pH 7.0), 100 mM NaCl, 3 microM oligonucleotides (n.d. means that any transition was not observed over 15 °C; the Tₘ is lower than 15 °C). The preferred implementations (A* = 2-aminopurine, T* = 2-thiothymine, C* = N⁴-ethylcytosine, G* = hypoxanthine) were used.

**Table 6.1 Tₘ values for Taq gene primers having different lengths**

| sequence | Tₘ (°C) |
|---|---|
| 5'-TGCCCTGTCTCTGGAGGT-3' SEQ ID NO 43 | 66.0 |
| 3'-ACGGGACAGAGACCTCCA-5' SEQ ID NO 44 | |
| 5'-T*G*C*C*C*T*G*T*C*T*C*T*G*G*A*G*G*T-3' SEQ ID NO 45 | 30.8 |
| 3'-A C G G G A C A G A G A C C T C C A-5' SEQ ID NO 44 | |
| 5'-T G C C C T G T C T C T G G A G G T -3' SEQ ID NO 43 | 26.0 |
| 3'-A C*G*G*G*A*C*A*G*A*G*A*C*C*T*C*C*A*-5' | |
| SEQ ID NO 46 | |
| 5'-T*G*C*C*C*T*G*T*C*T*C*T*G*G*A*G*G*T -3' SEQ ID NO 45 | n.d. |
| 3'-A C*G*G*G*A*C*A*G*A*G*A*C*C*T*C*C*A*-5' SEQ ID NO 46 | |
| 5'-TATCTGCGTGCCCTGTCTCTGGAGG-3' SEQ ID NO 31 | 74.0 |
| 3'-ATAGACGCACGGGACAGAGACCTCC-5' SEQ ID NO 32 | |
| 5'-T*A*T*C*T*G*C*G*T*G*C*C*C*T*G*T*C*T*C*T*G*G*A*G*G-3' SEQ ID NO 47 | 42.0 |
| 3'-A T A G A C G C A C G G G A C A G A G A C C T C C-5' SEQ ID NO 32 | |
| 5'-T A T C T G C G T G C C C T G T C T C T G G A G G-3' SEQ ID NO 31 | not meas. |
| 3'-A T*A*G*A*C*G*C*A*C*G*G*G*A*C*A*G*A*G*A*C*C*T*C*C*-5' SEQ ID NO 48 | |
| 5'-T*A*T*C*T*G*C*G*T*G*C*C*C*T*G*T*C*T*C*T*G*G*A*G*G -3' SEQ ID NO 47 | not meas |
| 3'-A T*A*G*A*C*G*C*A*C*G*G*G*A*C*A*G*A*G*A*C*C*T*C*C*-5' SEQ ID NO 48 | |

**Table 6.2. Tₘ values for RET transcript primers.**

| sequence | Tₘ (°C) |
|---|---|
| 5'-C*C*A*G*G*A*T*C*C*A*C*T*G*T*G*C*G*A*C*G*A*G*C*T*G -3' SEQ ID NO 33 | 40.3 |
| 3'-G G T C C T A G G T G A C A C G C T G C T C G A C -5' SEQ ID NO 34 | |
| 5'-C G C A C G G T G A T C G C A G C C G C T G T C C -3' SEQ ID NO 35 | 44.0 |
| 3'-G C*G*T*G*C*C*A*C*T*A*G*C*G*T*C*G*G*C*G*A*C*A*G*G*-5' SEQ ID NO 36 | |

These and other data lead to the heuristic rules that are taught in this application for the preferred components of SAMRS building blocks and their preferred placement in oligonucleotides, key to their utility as primers, and especially as primer pairs targeted at two positions in a target gene to support PCR, and in particular multiplexed PCR, where a plurality of pairs of primers has utility to solve the "multiplexed PCR problem".

### Example 7. Tₘ of primers targeted against Taq as a function of salt concentration.

To further develop these heuristic rules, especially with shorter 18mers (rather than 25mers) experiments were run to determine whether higher concentration of salt would stabilize duplexes that incorporate oligonucleotides containing SAMRS components, especially preferred components placed in those oligonucleotide in positions preferred to support PCR. Again, the very last nucleotide at the 3'-end was a standard nucleotide. Conditions: 3 microM oligonucleotides. n.d. means that any transition was not observed over 15 °C (Tₘ is lower than 15 °C.). A* = 2-aminopurine; T* = 2-thiothymine; C* = N⁴-ethylcytosine; G* = hypoxanthine. These experiments identified (at last) a behavior of SAMRS-containing oligonucleotides that is also found in natural DNA; Tₘ values were higher in higher salt concentration (1 M NaCl) than in lower salt (100 mM NaCl).

**Table 7.1. Melting temperature of 18mer pairs in low salt.**

| sequence | Tₘ (°C) |
|---|---|
| 5'-TGCCCTGTCTCTGGAGGT-3' SEQ ID NO 43 | 66.0 |
| 3'-ACGGGACAGAGACCTCCA-5' SEQ ID NO 44 | |
| 5'-T*G*C*C*C*T*G*T*C*T*C*T*G*G*A*G*G*T-3' SEQ ID NO 45 | 30.8 |
| 3'-A C G G G A C A G A G A C C T C C A-5' SEQ ID NO 44 | |
| 5'-T G C C C T G T C T C T G G A G G T -3' SEQ ID NO 43 | 26.0 |
| 3'-A C*G*G*G*A*C*A*G*A*G*A*C*C*T*C*C*A*-5' SEQ ID NO 46 | |
| 5'-T*G*C*C*C*T*G*T*C*T*C*T*G*G*A*G*G*T -3' SEQ ID NO 45 | n.d. |
| 3'-A C*G*G*G*A*C*A*G*A*G*A*C*C*T*C*C*A*-5' SEQ ID NO 46 | |

Conditions (low salt): 20 mM Na cacodylate (pH 7.0), 100 mM NaCl.

**Table 7.2. Melting temperature of 18mer pairs in high salt.**

| sequence | Tₘ (°C) | ΔTₘ (°C) |
|---|---|---|
| 5'-TGCCCTGTCTCTGGAGGT-3' SEQ ID NO 43 | 74.0 | 8.0 |
| 3'-ACGGGACAGAGACCTCCA-5' SEQ ID NO 44 | | |
| 5'-T*G*C*C*C*T*G*T*C*T*C*T*G*G*A*G*G*T-3' SEQ ID NO 45 | 36.0 | 5.2 |
| 3'-A C G G G A C A G A G A C C T C C A-5' SEQ ID NO 44 | | |
| 5'-T G C C C T G T C T C T G G A G G T-3' SEQ ID NO 43 | 30.5 | 4.5 |
| 3'-A C*G*G*G*A*C*A*G*A*G*A*C*C*T*C*C*A*-5' SEQ ID NO 46 | | |
| 5'-T*G*C*C*C*T*G*T*C*T*C*T*G*G*A*G*G*T -3' SEQ ID NO 45 | n.d. | -- |
| 3'-A C*G*G*G*A*C*A*G*A*G*A*C*C*T*C*C*A*-5' SEQ ID NO 46 | | |

Conditions (high salt): no 0 Na cacodylate (pH 7.0), 1 M NaCl.

### Example 8. Tₘ for alternative implementations of A* as a function of salt concentration.

To further develop heuristic rules and to define the metes and bounds of the preferred SAMRS systems, experiments were done to compare the Tₘ of duplexes with a single SAMRS pair embedded in a 15mer, with 2,6-diaminopurine replacing 2-amino purine as of A*. Conditions: 20 mM Na cacodylate (pH 7.0), 100 mM NaCl, 3 microM oligonucleotides. n.d. means that any transition was not observed over 15 °C (Tₘ is lower than 15 °C.). The Tₘ values were higher in higher salt concentration (1 M NaCl).

**Table 8.1. Sequences to compare 2-aminopurine with 2,6-diaminopurine as A***

| | |
|---|---|
| SAMRS-Tm1 : | 5'-ACCAAGCXATCAAGT-3' X=adenine SEQ ID NO 5 |
| SAMRS-Tm1 : | 5'-ACCAAGCXATCAAGT-3' X=2-aminopurine SEQ ID NO 6 |
| SAMRS-Tm1 : | 5'-ACCAAGCXATCAAGT-3' X=2-aminoadenine SEQ ID NO 117 |
| SAMRS-Tm1-C: | 3'-TGGTTCGYTAGTTCA-5' Y = T SEQ ID NO 15 |
| SAMRS-Tm1-C: | 3'-TGGTTCGYTAGTTCA-5' Y = 2-aminopurine SEQ ID NO 13 |
| SAMRS-Tm1-C: | 3'-TGGTTCGYTAGTTCA-5' Y = X=2-aminoadenine SEQ ID NO 118 |

**Table 8.2. Tₘ comparison of 2-aminopurine and 2,6-diaminopurine**

| X:Y | Tₘ (°C) | ΔTₘ (°C) | X:Y | Tₘ (°C) | ΔTₘ (°C) |
|---|---|---|---|---|---|
| A:T | 55.5 | -- | A:T* | 56.8 | +1.3 |
| A*:T | 54.5 | -1.0 | A*:T* | 52.0 | -2.5, -4.8 |
| DAP:T | 57.3 | +1.8 | DAP:T* | 52.3 | -5.0, -4.5 |
| T:A | 54.0 | -- | T*:A | 54.0 | ±0 |
| T:A* | 52.5 | -1.5 | T*:A* | 50.3 | -2.2, -3.7 |
| T:DAP | 56.5 | +1.0 | T*:DAP | 50.8 | -5.7, -3.2 |

### Example 9. Examination of Tₘ of 2-methylhypoxanthine (2MeI) as an alternative for hypoxanthine as a G*, and N⁴-ethylcytosine as an implementation of C*.

To further develop heuristic rules and to define the metes and bounds of the preferred SAMRS systems, experiments were done to compare an alternative implementation of G*; the methyl group of 2-methylinosine might, from other systems, be expected to stabilize the duplex. The indicated oligonucleotides were prepared and the melting temperatures were determined. Conditions: 20 mM Na cacodylate (pH 7.0), 100 mM NaCl, 3 microM oligonucleotides. n.d. means that any transition was not observed over 15 °C (Tm is lower).

**Table 9.1. Sequences to compare 2-methylhypoxanthine (2MeI) and N⁴-ethylcytosine**

| |
|---|
| 5'-ACCAAGCXATCAAGT-3' X= cytosine, SEQ ID NO 11 |
| 5'-ACCAAGCXATCAAGT-3' X= hypoxanthine, SEQ ID NO 10 |
| 5'-ACCAAGCXATCAAGT-3' X= 2-methylhypoxanthine, SEQ ID NO 119 |
| 3'-TGGTTCGYTAGTTCA-5' Y = thymine SEQ ID NO 15 |
| 3'-TGGTTCGYTAGTTCA-5' Y = 2-aminopurine SEQ ID NO 13 |
| 3'-TGGTTCGYTAGTTCA-5' Y = X=2-methylhypoxanthine, SEQ ID NO 120 |

**Table 9.2. Tₘ comparison of 2-methylhypoxanthine (2MeI) and N⁴-ethylcytosine**

| X:Y | Tₘ (°C) | ΔTₘ (°C) | X:Y | Tₘ (°C) | ΔTₘ (°C) |
|---|---|---|---|---|---|
| G*:C | 54.1 | --- | G*:N4eU | 52.0 | -2.1 |
| 2MeI:C | 51.3 | --- | 2MeI:N4eU | 48.0 | -3.3 |
| C:G* | 52.6 | --- | N4eU:G* | 51.5 | -1.1 |
| C:2MeI | 49.0 | --- | N4eU:2MeI | 46.0 | -3.0 |

Conditions: 20 mM Na cacodylate (pH 7.0), 100 mM NaCl, 3 µM each oligonucleotide. 2MeI represents 2-Me-inosine.

### Example 10. Examination of melting temperatures with N⁴-ethylcytosine (N4eU) as an implementation of C*.

To further develop heuristic rules, which requires information on mismatches between preferred SAMRS components and their standard, non complementary components, a series of melting temperatures were made for oligonucleotides containing single mismatches, here with N⁴-ethylcytosine. Conditions: 20 mM Na cacodylate (pH 7.0), 100 mM NaCl, 3 microM oligonucleotides. n.d. means that any transition was not observed over 15 °C.

**Table 10.1. Sequences to support heuristic rule. Mismatches with N⁴-ethylcytosine**

| |
|---|
| 5'-ACCAAGCXATCAAGT-3' X= a, SEQ ID NO 5 |
| 5'-ACCAAGCXATCAAGT-3' X= t, SEQ ID NO 7 |
| 5'-ACCAAGCXATCAAGT-3' X= g, SEQ ID NO 9 |
| 5'-ACCAAGCXATCAAGT-3' X= c, SEQ ID NO 11 |
| 5'-ACCAAGCXATCAAGT-3' X= N4-ethylcytosine, N4eU, SEQ ID NO 121 |
| 3'-TGGTTCGYTAGTTCA-5' Y = a SEQ ID NO 13 |
| 3'-TGGTTCGYTAGTTCA-5' Y = t SEQ ID NO 15 |
| 3'-TGGTTCGYTAGTTCA-5' Y = g SEQ ID NO 17 |
| 3'-TGGTTCGYTAGTTCA-5' Y = c SEQ ID NO 19 |
| 3'-TGGTTCGYTAGTTCA-5' Y = N4-ethylcytosine, N4eU, SEQ ID NO 114 |

**Table 10.2. Tₘ data to support heuristic rule. Mismatches with N⁴-ethylcytosine**

| X : Y | Tm (°C) | ΔTₘ(°C) | X:Y | Tm (°C) | ΔTₘ (°C) |
|---|---|---|---|---|---|
| A:N4eU | 45.3 | -10.4 | A*:N4eU | 46.8 | -8.9 |
| T:N4eU | 41.6 | -14.1 | T*:N4eU | 40.3 | -15.4 |
| G:N4eU | 55.7 | - | G*:N4eU | 52.0 | -3.7 |
| C:N4eU | 42.0 | -13.7 | N4eU:N4eU | 40.8 | -14.9 |
| | | | | | |
| N4eU:A | 43.0 | -12.8 | N4eU:A* | 45.0 | -10.8 |
| N4eU:T | 41.8 | -14.0 | N4eU:T* | 40.0 | -15.8 |
| N4eU:G | 55.8 | - | N4eU:G* | 51.5 | -4.3 |
| N4eU:C | 41.8 | -14.0 | N4eU:N4eU | 40.8 | -15.0 |

### Example 11. Examination of melting temperatures with mixed sequences with non-preferred implementation of C*.

Development of the preferred SAMRS systems for pairs of PCR primers (which involves the addition of standard nucleoside triphosphates to a primer containing SAMRS components), including preferred implementations of A*, T*, C*, and G*, was imperfectly guided by the literature that had been developed to support the addition of SAMRS triphosphates by polymerases to primers *not* containing SAMRS nucleotides (the opposite) to prepare non-structured nucleotides see [Gam06][Lah08]), develop complementary pairs (which pairs of primers useful for PCR are not) to invade duplexes (as in US Patent 5912340), or to use self-avoidance in processes that lack polymerases to do array-based oligonucleotide detection (see US Patent 7371580). Much of the issue in the literature concerned incomplete development of implementations of C* and G* (much of the early work focused on furano and pyrrolo fused ring systems; see [Woo96] and US Patent 7371580, a paucity of experiments to collect a critical mass of melting temperature data, and a lack of experiments to determine if polymerases could use SAMRS-containing primers, especially in PCR architectures, where they must also serve as templates.

To determine preferred implementations of C*, for example, many such experiments were run. Table 11.1 collects these for two implementations of C*, the most preferred N⁴-ethylcytosine and the less preferred 5-methyl-pyrimidin-2-one.

**Table 11.1. Tₘ with various implementations of C*. C* and eC represent 5-methyl-pyrimidin-2-one and N⁴-ethylcytosine, respectively.**

| sequence | Tm (°C) | ΔTm (°C) |
|---|---|---|
| 5'-TGCCCTGTCTCTGGAGGT-3' SEQ ID NO 43 | 66.0 | |
| 3'-ACGGGACAGAGACCTCCA-5' SEQ ID NO 44 | | |
| 5'-TATTTTATTTTTAAAAAT-3' SEQ ID NO 49 | 39.1 | |
| 3'-ATAAAATAAAAATTTTTA-5' SEQ ID NO 50 | | |
| 5'-TGC C C TGTC TC TGGAGGT-3' SEQ ID NO 43 | 50.4 | |
| 3'-ACG*G*G*ACAG*AG*ACCTCCA-5' SEQ ID NO 55 | | 15.6 |
| 5'-TGC*C*C*TGTC*TC*TGGAGGT-3' SEQ ID NO 51 | 37.3 | |
| 3'-ACG G G ACAG AG ACCTCCA-5' SEQ ID NO 44 | | 28.7 |
| 5'-TGC*C*C*TGTC*TC*TGGAGGT-3' SEQ ID NO 51 | 26.0 | |
| 3'-ACG*G*G*ACAG*AG*ACCTCCA-5' SEQ ID NO 55 | | 40.0 |
| 5'-TGeCeCeCTGTeCTeCTGGAGGT-3' SEQ ID NO 52 | 55.2 | |
| 3'-AC G G GACA GA GACCTCCA-5' SEQ ID NO 44 | | 10.8 |
| 5'-TGeCeCeCTGTeCTeCTGGAGGT-3' SEQ ID NO 52 | 40.0 | |
| 3'-ACG*G*G*ACAG*AG*ACCTCCA-5' SEQ ID NO 55 | | 26.0 |

Conditions: 20 mM Na cacodylate (pH 7.0), 100 mM NaCl, 3 microM oligonucleotides. n.d. means that any transition was not observed over 15 °C.

### Example 12. Examination of melting temperatures with mixed sequences with non-preferred implementation of C*.

Further experiments were run to explore a range of alternative implementations or C*, shown in Fig. 11. Conditions: 20 mM Na cacodylate (pH 7.0), 100 mM NaCl, 3 microM oligonucleotides. n.d. means that any transition was not observed over 15 °C.

**Table 12.1. Sequences to explore alternative versions of C* (see also Fig. 11)**

| |
|---|
| 5'-ACCAAGCXATCAAGT-3' X= a, SEQ ID NO 5 |
| 5'-ACCAAGCXATCAAGT-3' X= t, SEQ ID NO 7 |
| 5'-ACCAAGCXATCAAGT-3' X= g, SEQ ID NO 9 |
| 5'-ACCAAGCXATCAAGT-3' X= c, SEQ ID NO 11 |
| 5'-ACCAAGCXATCAAGT-3' X= h, SEQ ID NO 10 |
| 5'-ACCAAGCXATCAAGT-3' X= 4-methylpyrimidin-2-one, 4mC*, SEQ ID NO 122 |
| 5'-ACCAAGCXATCAAGT-3' X= N4-methylcytosine, N4mU, SEQ ID NO 123 |
| 5'-ACCAAGCXATCAAGT-3' X= N4-methyl-5-methylcytosine, N4mT, SEQ ID NO 124 |
| 3'-TGGTTCGYTAGTTCA-5' Y = a SEQ ID NO 13 |
| 3'-TGGTTCGYTAGTTCA-5' Y = t SEQ ID NO 15 |
| 3'-TGGTTCGYTAGTTCA-5' Y = g SEQ ID NO 17 |
| 3'-TGGTTCGYTAGTTCA-5' Y = c SEQ ID NO 19 |
| 3'-TGGTTCGYTAGTTCA-5' Y = h SEQ ID NO 18 |
| 3'-TGGTTCGYTAGTTCA-5' Y = 4-methylpyrimidin-2-one, 4mC*, SEQ ID NO 111 |
| 3'-TGGTTCGYTAGTTCA-5' Y = N4-methylcytosine, N4mU, SEQ ID NO 113 |
| 3'-TGGTTCGYTAGTTCA-5' Y = N4-methyl-5-methylcytosine, N4mT, SEQ ID NO 115 |

**Table 12.1. Melting temperatures with mixed sequences with various implementations of C* (see Fig. 11 for abbreviations).**

| X:Y | Tm (°C) | ΔTm (°C) | X:Y | Tm (°C) | ΔTm (°C) |
|---|---|---|---|---|---|
| G:C | 58.8 | --- | G:C* | 52.0 | -6.8 |
| G:4mC* | | | G:45dmC* | 51.0 | -7.8 |
| G:N4mU | 57.0 | -1.8 | G:N4eU | 55.7 | -3.1 |
| G:N4mT | 46.5 | -12.3 | G:N4eT | | |
| G*:C | 54.1 | --- | G*:C* | 49.3 | -4.8 |
| G*:4mC* | | | G*:45dmC* | 49.8 | -4.3 |
| G*:N4mU | 53.8 | -0.3 | G*:N4eU | 52.0 | -2.1 |
| G*:N4mT | 45.8 | -8.3 | G*:N4eT | | |
| A:T | 55.5 | --- | | | |
| | | | | | |
| C:G | 59.0 | --- | C*:G | 52.0 | -7.0 |
| 4mC*:G | | | 45dmC*:G | | |
| N4mU:G | 56.3 | -2.7 | N4eU:G | 55.8 | -3.2 |
| N4mT:G | 45.8 | -13.2 | N4eT:G | | |
| C:G* | 52.6 | --- | C*:G* | 47.8 | -4.8 |
| 4mC*:G* | | | 45dmC*:G* | | |
| N4mU:G* | 51.3 | -1.3 | N4eU:G* | 51.5 | -1.1 |
| N4mT:G* | 45.5 | -7.1 | N4eT:G* | | |
| T:A | 54.0 | --- | | | |

These results show heuristic rules that N⁴-Et-C:G contributes like T:A pair [Ngu00], while the N⁴-Et-C seems to prefer G to G* (55.7 vs. 52.0 and 55.8 vs. 51.5). G* = hypoxanthine.

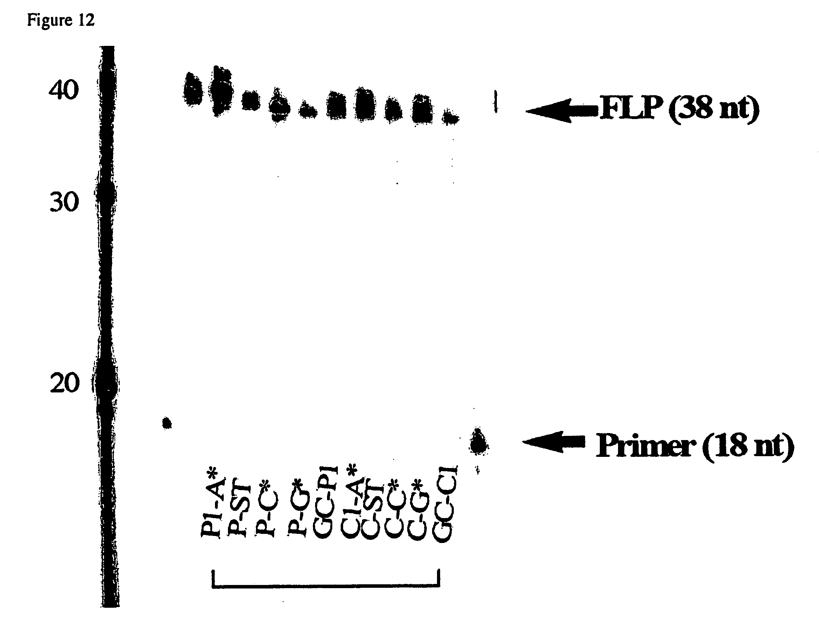

### Example 13. Primer extensions using primers with scattered SAMRS components (Fig. 12)

To develop heuristic rules to predict duplex stability and suitability as primers for oligonucleotides containing SAMRS components, a series of primer extension experiments were run. The 5-methyl zebularine derivative (where the heterocycle implementing a C* hydrogen bonding pattern is 5-methylpyrimidin-2-one) was found to perform sufficiently well that polymerases were sought that accept it and other SAMRS analogs in primers and templates. This example shows that the following set of SAMRS implementations are acceptable, to a level, with a heuristic rule that T*>A*>*C*>G*, in order from easy to difficult. See Figure 12 for results. The implementations were: A* = 2-aminopurine, T* = 2-thiothymine, C* = 5-methyl-pyrimidin-2-one, G* = hypoxanthine
Templates:
   Template 1: 3'-ACGGGACAGAGACCTCCAACGACTTCTTTAGCGCGCAG 5' SEQ ID NO 60
   Template 2: 5'-CTGGATGTTGCATATCTGCGTGCCCTGTCTCTGGAGGT 3' SEQ ID NO 61
   Reverse Primer 1: 3'GACTTCTTTAGCGCGCAG 5' SEQ ID NO 62
   Reverse Primer 2: 5'CTGGATGTTGCATATCTG 3' SEQ ID NO 63
Forward Primer:
   C-Series:
      C1-A^{*}: 3'-A CGGGA*CA*GA*GA*CCTCCA*-5' SEQ ID NO 64
      C-ST: 3'-ACGGGACAGAGACCT*CCA-5' SEQ ID NO 65
      C-C*: 3'-AC*GGGAC*AGAGAC*C*TC*C*A-5' SEQ ID NO 66
      C-G*: 3'-ACG*G*G*ACA G*AG*ACCTCCA-5' SEQ ID NO 55
      GC-C1: 3'-AC*G*G*G*AC*AG*AG*AC*C*TC*C*A-5' SEQ ID NO 67
   P-Series:
      P-A*: 5'-TGCCCTGTCTCTGGA*GGT-3' SEQ ID NO 68
      P-ST: 5'-T*GCCCT*GT*CT*CT*GGAGGT-3' SEQ ID NO 69
      P-C*: 5'-TGC*C*C*TGTC*TC*TGGAGGT-3' SEQ ID NO 52
      P-G*: 5'-TG*CCCTG*TCTCTG*G*AG*G*T-3' SEQ ID NO 70
      GC-P1:5'-TG*C*C*C*TG*TC*TC*TG*G*AG*G*T-3' SEQ ID NO 71
Template/Primer complex:
   P-Series: 5'TGCCCTGTCTCTGGAGGT-3' SEQ ID NO 72
      Template 1: 3' ACGGGACAGAGACCTCCAACGACTTCTTTAGCGCGCAG 5' SEQ ID NO 73
   C-Series: 3'ACGGGACAGAGACCTCCA 5' SEQ ID NO 44
      Template 2: 5'- CTGGATGTTGCATATCTGCGTGCCCTGTCTCTGGAGGT-3' SEQ ID NO 61

SAMRS primers (18 nt) were annealed to the complementary template by heating (2 min) at 95 °C followed by slow cooling to 20 °C (over 20 min). Alpha-³²P-dCTP and dNTPs was then added, followed by Klenow fragment of DNA polymerase 1. Incubation was continued for various times at indicated temperatures, and quenched with 10 mM EDTA in formamide loading buffer. The resulting reaction mixtures were separated on 14 % PAGE and visualized by autoradiography. The results are shown in Figure 12.

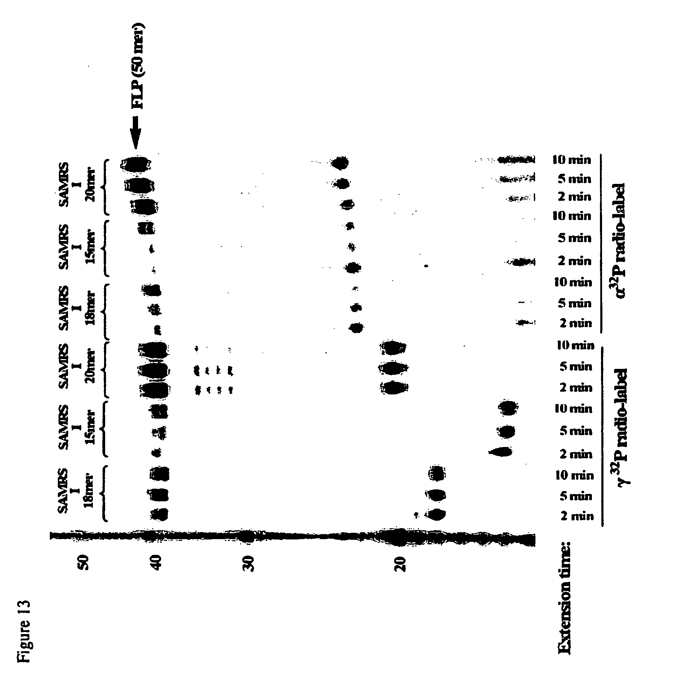

### Example 14. Primer extensions using primers with all SAMRS components (except 3'-end) (Figure 13)

Polymerase data were obtained with the following implementation: A* = 2-aminopurine, T* = 2-thiothymine, C* = 5-methylpyrimidin-2-one, G* = hypoxanthine, and the following sequences:
32P SAMRS I 15mer: 5'-T*G*C*C*C*T*G*T*C*T*C*T*G*G*A -3'. SEQ ID NO 75
SAMRS I 18mer: 5'-T*G*C*C*C*T*G*T*C*T *C*T*G*G*A*G*G*T-3' SEQ ID NO 45
SAMRS I 20mer: 5'-T*G*C*C*C*T*G*T*C*T*C*T*G*G*A*G*G*T*T*G-3' SEQ ID NO 75
Template: 3' ACGGGACAGAGACCTCCAACGACTTCTTTAGCGCGCAG 5' SEQ ID NO 61

SAMRS I 20mer, SAMRS I 18mer and SAMRS I 15mer were elongated on a standard template by using Klenow (exo-) in the presence extra 50 mM NaCl. The sequences were shown as above. γ 32P-labeled 20mer, 18mer and 15mer of SAMRS primers were annealed to the complementary template first. dNTPs (100µM, final) were then added, followed by Klenow (exo-) polymerase. The reactions were performed at 30 °C. At 2 min, 5 min and 10 min, aliquots of reaction mixtures were taken and quenched with 10 mM EDTA in formamide loading buffer. The resulting reaction mixtures were separated on 10 % PAGE and visualized by autoradiography Fig. 13). Internal radiolabeling experiments were also carried out in parallel. Cold 20 mer, 18me and 15mer of SAMRS primers were annealed to the complementary template first. Alpha 32P -dCTP. dNTPs were then added, followed by Klenow (exo-) polymerase. The other reaction conditions were identical to above. The Figure makes clear why these implementations are not presently preferred

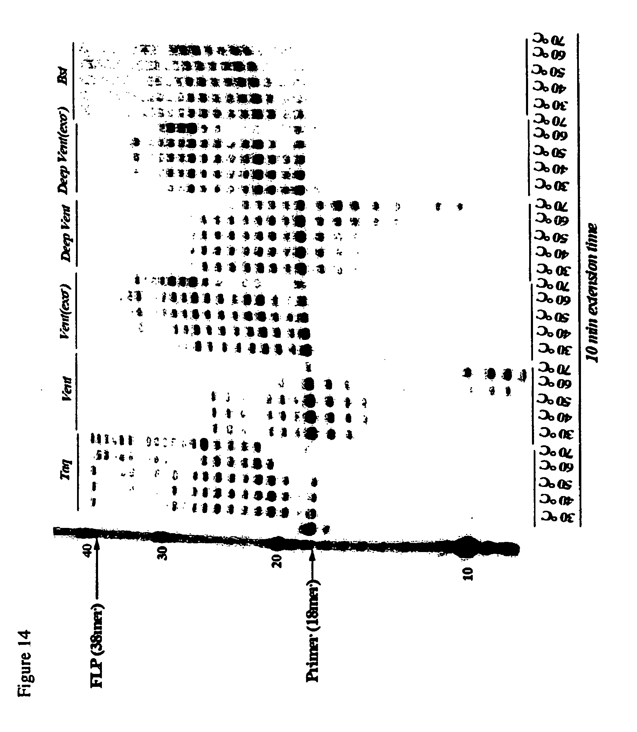

### Example 15. Polymerase read-through of templates containing consecutive SAMRS components (Figure 14)

This experiment demonstrates the level of read through of templates containing SAMRS components using 6 kinds of NEB thermophilic polymerases at various temperatures. The following template-primer combination was used.
5'-A*C*G*A*C*T*G*G*G*T*T*T*C*C*A*A*G*G*GG CTGAAGAAATCGCGCGTC-3' SEQ ID NO 76
3'- GAC TTC TTT AGC GCG CAG -5' SEQ ID NO 62

γ-³²P-labeled primer was annealed to the complementary SAMRS template as above, and then the annealed template-primer complex was contacted with dNTPs in solution (100 µM, final concentration), followed by 6 kinds of NEB thermophilic polymerases as indicated. The reactions were performed at 30 °C, 40 °C, 50 °C, 60 °C and 70 °C for 10 min, and aliquots of reaction mixtures were taken and quenched with 10 mM EDTA in formamide loading buffer. The resulting reaction mixtures were separated on 14 % PAGE and visualized by auto radiography.

Results: Six different thermophilic polymerases were initially screened for their ability to read through SAMRS in template. Surprisingly and unexpectedly, Figure 1 showed that the 6 NEB thermophilic polymerases were all able to read through consecutive SAMRS components in templates to some extent. Taq, vent (exo-), Deep Vent (exo-) and Bst DNA polymerases showed better ability to read through the SAMRS template. Taq DNA polymerase performed the best. Also, the read-through full length product increased with increase in the incubation temperature. Since Vent and Deep Vent polymerases contain 3' to 5' exonuclease activity, the hot primers were degraded and the small successive bands were shown on the gel. Taq and Bst DNA polymerases are therefore the preferred polymerases for reading templates containing SAMRS components.

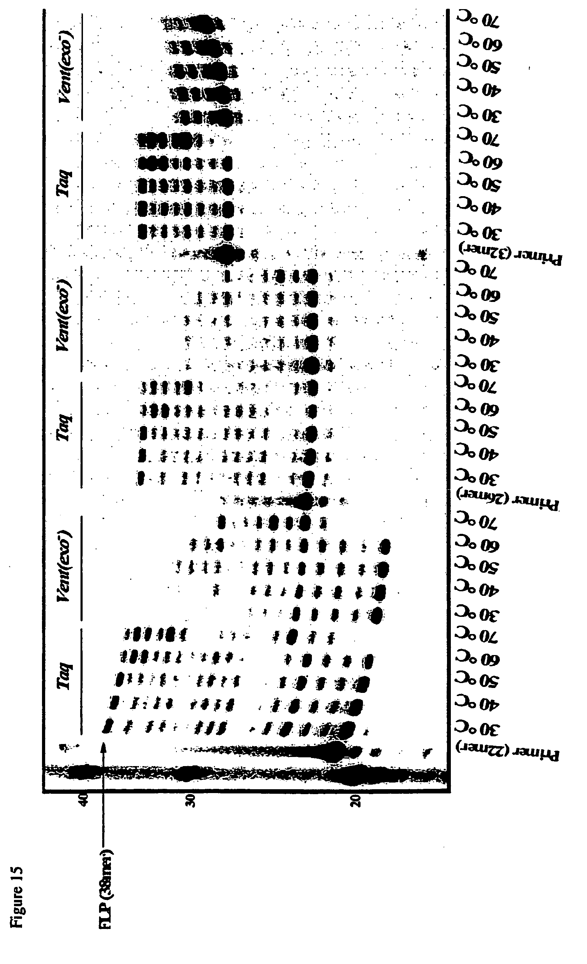

### Example 16. Read through of thioT in templates (Figure 15)

This experiment demonstrates the level of read through of templates containing SAMRS components with the following implementations: A* = 2-aminopurine, T* = 2-thiothymine, C* = 5-methylpyrimidin-2-one, G* = hypoxanthine. Taq and Vent (exo-) were then examined to identify difficulties in reading through T* in a template by using the longer primers and various concentrations of dNTPs. The following synthetic templates and primers were used.
5'-A*C*G*A*C*T*G*G*G*T*T*T*C*C*A*A*G*G*GGCTGAAGAAATCGCGCGTC-3' SEQ ID NO 77
Primer 2: 3'- C C CC GAC TTC TTT AGC GCG CAG -5' SEQ ID NO 78
5'-A*C*G*A*C*T*G*G*G*T*T*T*C*C*A*A*G*G*GGCTGAAGAAATCGCGCGTC-3' SEQ ID NO 77
Primer 3: 3'- G G T T C C CC GAC TTC TTT AGC GCG CAG-5' SEQ ID NO 79
5'-A*C*G*A*C*T*G*G*G*T*T*T*C*C*A*A*G*G*GG CTGAAGAAATCGCGCGTC-3' SEQ ID NO 77
Primer 4: 3' - C C C A A A G G T T C C CC GAC TTC TTT AGC GCG CAG -5' SEQ ID NO 80

Three γ-³²P-labeled primers were annealed to the complementary SAMRS template, dNTPs were then added (100 µM, final), followed by Taq and vent (exo-) polymerases as indicated. The reactions were performed at 30 °C, 40 °C, 50 °C, 60 °C and 70 °C for 10 min, and aliquots of reaction mixtures were taken and quenched with 10 mM EDTA in formamide loading buffer. The products were separated (14% PAGE) and visualized by autoradiography (Fig. 14).

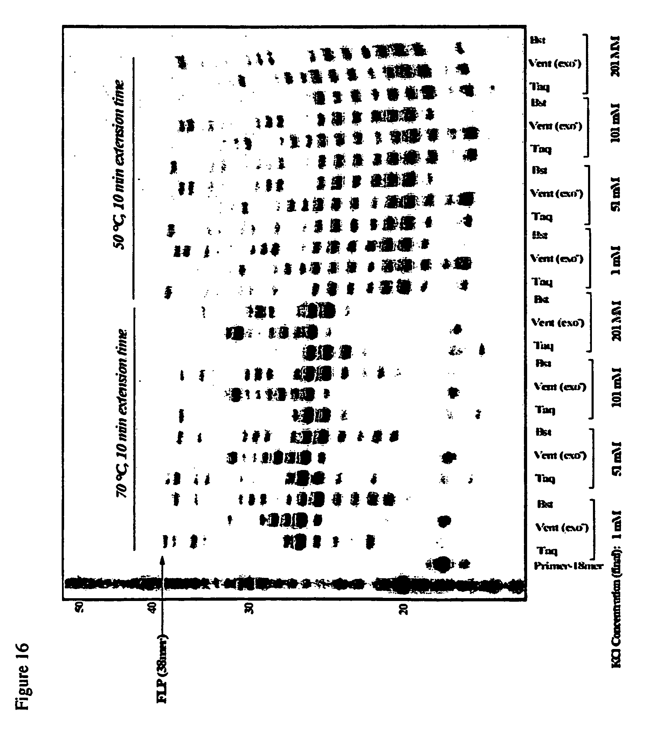

### Example 17. Readthrough of SAMRS in templates at different KCl concentrations (Fig. 16)

This experiment optimized conditions of polymerase read-through of the SAMRS-containing template by varying the concentrations of KCl, so as to decrease the pausing during the read through with the non-preferred implementation of C*. Three thermophilic polymerases were used together with the following
5'-A*C*G*A*C*T*G*G*G*T*T*T*C*C*A*A*G*G*GGCTGAAGAAATCGCGCGTC-3'
   C* = N4-ethylcytosine SEQ ID NO 76
C* = 5-methyl-pyrimidin-2-one SEQ ID NO 77
3'- GAC TTC TTT AGC GCG CAG -5' SEQ ID NO 62

γ-³²P-labeled primer was annealed to the complementary SAMRS template as described above. dNTPs were then contacted in aqueous solution added (100 µM, final concentration of each), followed by three NEB thermophilic polymerases as indicated. The reactions were performed at 50 °C and 70 °C for 10 min and quenched with 10 mM EDTA in formamide loading buffer. Products were separated (14 % PAGE, autoradiography). The results show that increasing the concentration of KCl decreased pausing (Fig. 16) with this implementation of C*.

### Example 18. PCR using SAMRS primers, with comparison to standard primers, targeted against 13 cancer genes (Figure 8 and Figure 9).

Examples above provide experimental data showing polymerases that support PCR with SAMRS primer pairs, the SAMRS components preferred to support PCR amplifications, and the placement of those in oligonucleotides to give the preferred sequences. Experiments were then done to demonstrate multiplexing of PCR using primers that contain SAMRS components. In this example, DNA targets are chosen to be of interest to cancer biologists. The targets were found in human genome DNA (Promega, 200 nM). The ratio of primer to target is high enough to be useful in PCR, and may also be used to define the metes and bounds of claims.
The following chimeric primers were used in a 16 + 8 + 1 format (16 standard nucleotides followed by 8 SAMRS nucleotides followed by 1 standard nucleotides).

**Table 18.1. Sequences of primers in the primer libraries**

| |
|---|
| RET-52-F: 5'-TTAGGGTCGGATTCCAG*T*T*A*A*A*T*G*G-3' SEQ ID NO 81 |
| RET-52-R: 3'-CC*G*T*T*A*A*C*T*TAGGGAAAAACTAGTA-5' SEQ ID NO 82 |
| FLT3-74-F: 5'-CGGGAAAGTGGTGAAGA*T*A*T*G*T*G*A*C-3' SEQ ID NO 83 |
| FLT3-74-R: 3'-TC*A*C*T*A*A*G*G*TTGATACAACAGTCCC-5' SEQ ID NO 84 |
| KIT-90-F: 5'-AGATTTGTGATTTTGGT*C*T*A*G*C*C*A*G-3' SEQ ID NO 85 |
| KIT-90-R: 3'-CA*C*T*C*A*T*G*G*GTAAGAGACGAACTGT-5 SEQ ID NO 86 |
| TSHR-131-F: 5'-CCGCAGTACAACCCAGG*G*G*A*C*A*A*A*G-3' SEQ ID NO 87 |
| TSHR-131-R: 3'-GT*C*G*T*T*A*A*G*ACTTGTTCGGAGAGTA-5' SEQ ID NO 88 |
| PDGFRA-152-F: 5'-GCTCGCAACGTCCTCCT*G*G*C*A*C*A*A*G-3' SEQ ID NO 89 |
| PDGFRA-152-R: 3'-GT*C*C*G*A*G*T*A*GGAGGAAGTGAAATTA-5' SEQ ID NO 90 |
| EGFR-176-F: 5'-CACAGCAGGGTCTTCTC*T*G*T*T*T*C*A*G-3' SEQ ID NO 91 |
| EGFR-176-R: 3'-CT*C*T*T*T*C*T*T*ATGGTACGTCTTCCTC-5' SEQ ID NO 92 |
| JAK2-203-F: 5'-CTGAAAGTAGGAGAAAG*T*G*C*A*T*C*T*T-3' SEQ ID NO 93 |
| JAK2-203-R: 3'-AG*A*C*A*C*C*T*C*TGCTCTCATTCATTTT-5' SEQ ID NO 94 |
| PIK3CA-233-F: 5'-TATTCGACAGCATGCCA*A*T*C*T*C*T*T*C-3' SEQ ID NO 95 |
| PIK3CA-233 -R: 3'-GT*G*T*G*T*T*A*A*TTTGTCGTACGTAACT-5' SEQ ID NO 96 |
| CTNNB1-258-F: 5'-CTAATACTGTTTCGTAT*T*T*A*T*A*G*C*T-3' SEQ ID NO 97 |
| CTNNB1-258-R: 3'-GA*G*T*T*C*T*T*G*TTCATCGACCATTCTC-5' SEQ ID NO 98 |
| PTPN11-309-F: 5'-AATAAAGACCTTTGTGT*T*G*A*G*T*T*G*G-3' SEQ ID NO 99 |
| PTPN11-309-R: 3'-CA*A*C*C*A*G*G*T*CATAATGTACCTTGTA-5' SEQ ID NO 100 |
| NRAS-336-F: 5'-CCATATTTCTTTTCTGC*A*G*G*C*A*T*A*T-3' SEQ ID NO 101 |
| NRAS-336-R: 3'-CT*T*C*T*C*A*T*G*TCACGGTACTCTCTGG-5' SEQ ID NO 102 |
| ABL1-470-F: 5'-CGGGAGCCCCCGTTCTA*T*A*T*C*A*T*C*A-3' SEQ ID NO 103 |
| ABL1-470-R: 3'-TT*C*A*C*C*T*T*A*TAATTTACTTCAAGTA-5' SEQ ID NO 104 |
| NRAS (II)-512-F: 5'-GTACAAACTGGTGGTGG*T*T*G*G*A*G*C*A-3' SEQ ID NO 105 |
| NRAS (II)-512-R: 3'-TC*G*A*T*C*A*A*C*TTCGTCGACTCTGGTC-5' SEQ ID NO 106 |
| APC-552-F: 5'-GTTCATTATCATCTTTG*T*C*A*T*C*A*G*C-3' SEQ ID NO 107 |
| APC-552-R: 3'-CT*T*C*A*T*G*G*A*TTTTTATTTCGTGGAT-5' SEQ ID NO 108 |

For Fig. 8, showing monoplexed PCR experiments with standard primers (Fig. 8a) and SAMRS chimeric primers (Fig. 8b), SAMRS was implemented as follows: T* = 2-thiothymine; A* = 2-aminopurine; G* = hypoxanthine; C* = N⁴-ethylcytosine. The template was human genomic DNA, 25 ng/ 25 microL Primers were each 200 nM, a concentration that is within the presently preferred range of greater than 100 nM. Concentration of dNTPs was each 0.2 mM. For standard primers, MgCl₂ concentration in commercially supplied Taq buffer was used (ca. 2 mM). For SAMRS primers, an additional 5 mM MgCl₂ was added. Taq polymerase: 1.0 units/ 0.025 mL. 40 cycles: denature at 94 °C for 1 min, annealing at 55 °C for SAMRS and 60 °C for standard primers for 1 min; then primer extension at 72 °C for 90 sec. Products were resolved on a 3% agarose gel and visualized by phosphorimager (one primer was 5'-radiolabeled).

For Fig. 9, showing five- and ten-fold multiplexed PCR experiments with standard primers (Fig. 9a) and SAMRS chimeric primers (Fig. 9b), with SAMRS hydrogen bonding patterns implemented as follows: T* = 2-thiothymine; A* = 2-aminopurine; G* = hypoxanthine; C* = N4-ethylcytosine. The template was human genomic DNA, 25 ng/25 microL Primers each 200 nM. dNTPs were each 1 mM. For standard primers, an additional 2.5 mM MgCl₂ was added to Taq buffer. For SAMRS primers, an additional 10 mM MgCl₂ was added. Taq polymerase: 5.0 units/0.025 mL. 40 cycles: denature at 94 °C for 1 min, then annealing at 55 °C for SAMRS and 60 °C for standard primers for 1 min; then primer extension at 72 °C for 90 sec. Products were resolved (3% agarose gel, phosphorimager (one primer was 5'-radiolabeled).

In this experiment, the selection of primers was driven by their biological interest; no effort was made to optimize the primer sequences, something that is expensive. It should be noticed that with standard uniplexed PCR (Fig. 8a), most amplicons were seen; with SAMRS uniplexed PCR (Fig. 8b), all amplicons were seen. With SAMRS primers, all of the amplicons were also seen in multiplexing (Fig. 9b). In contrast, with standard primers, some of the amplicons were lost upon multiplexing (Fig. 9a). This example therefore demonstrates the utility of SAMRS components, in their preferred form as taught here, and placed at positions in oligonucleotides as taught here, and delivered at the concentrations taught here, to support multiplexed PCR.

### References

- [A1198a]: Allawi, H. T., SantaLucia, J. (1998) Nearest-neighbor thermodynamics of internal A.C mismatches in DNA: Sequence dependence and pH effects. Biochemistry 37, 9435-9444
- [A1198b]: Allawi, H. T., SantaLucia, J. (1998) Thermodynamics of internal C:T mismatches in DNA. Nucleic Acids Res. 26, 2694-2701.
- [Ben04]: Benner, S. A. (2004) Understanding nucleic acids using synthetic chemistry. Acc. Chem. Res. 37, 784-797.
- [Bog99]: Bogan, J., Ignatovich, L., Stankevich,E. (1999) Reversed (5'->3') oligonucleotide synthesis on oxalyl-CPG support. Nucleosides Nucleotides Nucl. Acids 18, 1183-1185.
- [Bro97]: Brownie, J., Shawcross, S., Theaker, J., Whitcombe, D., Ferrie, R., Newton, C., Little, S. (1997). The elimination of primer-dimer accumulation in PCR. Nucleic Acids Res. 25, 3235-3241 1
- [Co197]: Collins,M.L., Irvine,B., Tyner,D., Fine,E., Zayati,C., Chang,C.A., Horn,T., Ahle,D., Detmer,J., Shen,L.P., Kolberg,J., Bushnell,S., Urdea,M.S., Ho,D.D. (1997) A branched DNA signal amplification assay for quantification of nucleic acid targets below 100 molecules/mL. Nucl. Acids Res. 25, 2979-2984.
- [Egh92]: Egholm, M., Buchardt, O., Nielsen, P. E., Berg, R. H. (1992) Peptide nucleic-acids (PNA) ; Oligonucleotide analogs with an achiral peptide backbone J. Am. Chem. Soc. 114, 1895-1897
- [Elb04a]: Elbeik, T., Markowitz, N., Nassos, P., Kumar, U., Beringer, S., Haller, B. and Ng, V. (2004) Simultaneous runs of the Bayer VERSANT HIV-1 version 3.0 and HCV bDNA version 3.0 quantitative assays on the system 340 platform provide reliable quantitation and improved work flow. J. Clin. Microbiol., 42, 3120-3127.
- [Elb04b]: Elbeik, T., Surtihadi, J., Destree, M., Gorlin, J., Holodniy, M., Jortani, S.A., Kuramoto, K., Ng, V., Valdes, R., Valsamakis, A. et al. (2004) Multicenter evaluation of the performance characteristics of the Bayer VERSANT HCV RNA 3.0 assay (bDNA). J. Clin. Microbiol., 42, 563-569.
- [Fre07]: Fredriksson, S., Baner, J., Dahl, F., Chu, A Ji, H., Welch, K., Davis, R. W. (2007) Multiplex amplification of all coding sequences within 10 cancer genes by Gene-Collector. Nucl. Acids Res. 35, e47.
- [Gam04]: Gamper, H. B., Gewirtz, A., Edwards, J., et al. (2004) Modified bases in RNA reduce secondary structure and enhance hybridization. Biochemistry 43, 10224-10236.
- [Gam06]: Gamper, H. B., Arar, K., Gewirtz, A., Hou, Y.-M. (2006) Unrestricted hybridization of oligonucleotides to structure-free DNA. Biochemistry 45, 6978-6986.
- [Gey03]: Geyer, C. R., Battersby, T. R., Benner, S. A. (2003) Nucleobase pairing in expanded Watson-Crick like genetic information systems. The nucleobases. Structure 11, 1485-1498.
- [Kot93]: Kotler,L.E., Zevin-Sonkin,D., Sobolev,I.A., Beskin, A.D., Ulanovsky,L.E. (1993) DNA sequencing: Modular primers assembled from a library of hexamers or pentamers. Proc. Natl. Acad. Sci. 90, 4241-4245.
- [Kut96]: Kutyavin, I. V., et al. (1996) Oligonucleotides containing 2-aminoadenine and 2-thiothymine act as selectively binding complementary agents Biochemistry 35, 11170-11176
- [Lah08a]: Lahoud, G., Timoshchuk, V., Lebedev, A., de Vega, M., et al. (2008) Properties of pseudo-complementary DNA substituted with weakly pairing analogs of guanine or cytosine. Nucl. Acids Res. 36, 6999-7008.
- [Lah08b]: Lahoud, G., Timoshchuk, V., Lebedev, A., de Vega, M., et al. (2008) Enzymatic synthesis of structure-free DNA with pseudo-complementary properties. Nucl. Acids Res. 36, 3409-3419.
- [Lah08c]: Loud, G. et al. (2008) RecA-mediated strand invasion of DNA by oligonucleotides substituted with 2-aminoadenine and 2-thiothymine. Nucleic Acids Res. 36, 6806-6815
- [Lan00]: Lan, T., McLaughlin, L.W. (2000) Minor groove hydration is critical to the stability of DNA duplexes. J. Am. Chem. Soc. 122,6512-6513.
- [Lea06]: Leal, N.A., Sukeda, M., Benner, S.A. (2006) Dynamic assembly of primers on nucleic acid templates. Nucleic Acids Res 34, 4702-4710.
- [Mar85]: Martin,F.H., Castro,M.M., Aboul-ela,F., Tinoco,I. Jr. (1985) Base-pairing involving deoxyinosine - implications for probe design. Nucl. Acids Res. 13, 8927-8938.
- [Mat98]: Mathews,D.H., Andre,T.C., Kim,J., Turner,D.H., Zuker,M. (1998) An updated recursive algorithm for RNA secondary structure prediction with improved thermodynamic parameters. Molecular Modeling of Nucleic Acids. ACS Symposium Series 682, 246-257.
- [Ngu00]: Nguyen, H.-K, Southern, E. M. (2000) Minimising the secondary structure of DNA targets by incorporation of a modified deoxynucleoside. Implications for nucleic acid analysis by hybridization. Nucl. Acids Res. 28, 3904-3909.
- [Ngu98]: Nguyen, H.-K. et al. (1998) The stability of duplexes involving AT and/or G4EtC base pairs is not dependent on their raio content. Implication for DNA sequencing by hybridization. Nucl. Acids Res. 28, 3904-3909.
- [Ono87]: Ono A, Ueda T. (1987) Nucleic Acids Res. 15, 219-s32.
- [San98]: SantaLucia, J. (1998) A unified view of polymer, dumbbell, and oligonucleotide DNA nearest-neighbor thermodynamics. Proc. Nat. Acad. Sci. USA 95, 1460-1465
- [Si199]: Silverman,B.D., Pitman,M.C., Platt,D.E.(1999) Molecular moment similarity between several nucleoside analogs of thymidine and thymidine. Journal of Biomolecular Structure & Dynamics 16, 1169-1175.
- [Sin01]: Singleton,S.F., Shan,F., Kanan,M.W., McIntosh,C.M., Stearman,C.J., Heim,J.S., Webb,K.J. (2001) Facile synthesis of afluorescent deoxycytidine analog suitable for probing the recA nucleoprotein filament. Org. Lett. 3919-3922.
- [Sis05]: Sismour,A.M., Benner,S.A. (2005) The use of thymidine analogs to improve the replication of an extra DNA base pair: A synthetic biological system. Nucl. Acids Res. 33, 5640-5646.
- [Stu89]: Studier,F.W. (1989) A strategy for high-volume sequencing of cosmid DNAs: random and directed priming with a library of oligonucleotides. Proc. Natl. Acad. Sci. USA 86, 6917-6921.
- [Szy90]: Szybalski,W. (1990) Proposal for sequencing DNA using ligation of hexamers to generate sequential elongation primers (SPEL-6). Gene 90, 177-178.
- [Viv04]: Vives,M., Eritja,R., Tauler,R., Marquez,V. E., Gargallo, R.(2004) Synthesis, stability, and protonation studies of a self-complementary dodecamer containing the modified nucleoside 2'-deoxyzebularine. Biopolymers 73, 27-43.
- [Woo03]: Woods, K. K., Lan, T., McLaughlin, L.W., Williams, L. D.(2003) The role of minor groove functional groups in DNA hydration. Nucleic Acids Research 31, 1536-1540.
- [Woo96]: Woo, J. Meyer, Jr., R. B., Gamper, H. B. (1996) G/C-modified oligodeoxynucleotides with selective complementarity: Synthesis and hybridization properties Nucl, Acids Res, 24,2470-2475
- [Zha01]: Zhan, Z.Y. J., Ye, J.D., Li, X.Y., Lynn, D.G. (2001) Replicating DNA differently. Current Org. Chem. 5, 885-902.

## Claims

1. A process for amplifying an oligonucleotide by enzymatic template-directed primed polymerization, wherein said process comprises (a) contacting in aqueous solution one or more pairs of oligonucleotide primers with said oligonucleotide, a polymerase, and standard nucleoside triphosphates, and (b) incubating the mixture for a preselected length of time, wherein each oligonucleotide primer in said pair has the formula: 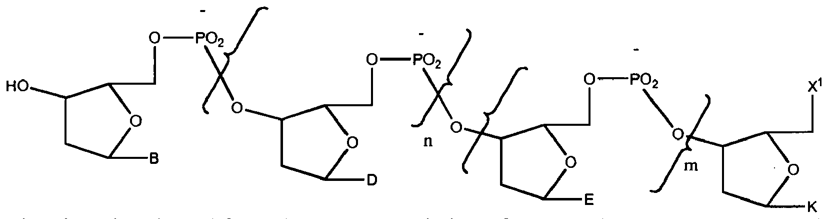 wherein X is selected from the group consisting of OH, O-phosphate, O-oligonucleotide, -NH₂, and a phosphate or an amino group linked to a biotin or flurorescent tag, B is independently selected from the group consisting of adenine, thymine, guanine, cytosine, diaminopurine, uracil, A*, T*, G*, and C*, D is independently selected from the group consisting of A*, T*, G*, and C*, E is independently selected from the group consisting of A, T, G, and C, and K is independently selected from the group consisting of A, T, G, C, A*, T*, G*, and C*, wherein n is an integer from 4 to 25 and m is an integer from 2 to 10, wherein the first member of said pair is complementary to said oligonucleotide at a portion of it, and the second member of said pair is formally the same as a segment of the same oligonucleotide at a position between 10 and 1000 nucleotides in the 5'-direction from the portion, where A* does not contribute to the stability of a duplex when paired with T* but does when it is paired with thymine, T* does not contribute to the stability of a duplex when paired with A* but does when it is paired with adenine, G* does not contribute to the stability of a duplex when paired with C* but does when it is paired with cytosine, and C* does not contribute to the stability of a duplex when paired with G* but does when it is paired with guanine, wherein A* is selected from the group consisting of 2-aminopurine and 2,6-diaminopurine, T* is selected from the group consisting of 2-thiothymine and 2-thiouracil, G* is hypoxanthine and C* is selected from the group consisting of N⁴-ethylcytosine and N⁴-methylcytosine.

2. The process of claim 1 wherein A* is selected from the group consisting of 2-aminopurine and 2,6-diaminopurine, T* is 2-thiothymine, G* is hypoxanthine and C* is selected from the group consisting of N⁴-ethylcytosine and N⁴-methylcytosine.

3. The process of claim 1 wherein the sum of m and n in said primers is at least 15.

4. The process of claim 1 wherein the D units are independently selected from the group consisting of T*, A*, G* and C*.

5. The process of claim 1 wherein the B units are independently selected from the group consisting of thymine, adenine, guanine, cytosine.

6. The process of claim 1 wherein more than 5 of said primer pairs are contacted.

7. A compound having the formula 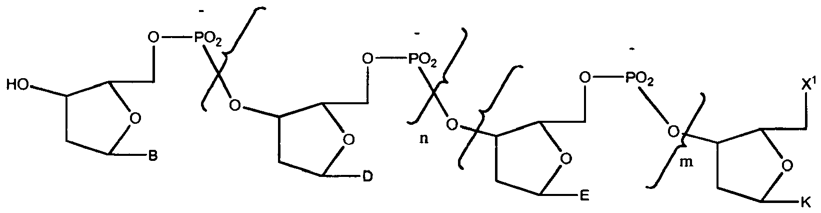 wherein X is selected from the group consisting of OH, O-phosphate, O-oligonucleotide, -NH₂, and a phosphate or an amino group linked to a biotin or fluorescent tag, B is independently selected from the group consisting of adenine, thymine, guanine, cytosine, diaminopurine, uracil, A*, T*, G*, and C*, D is independently selected from the group consisting of A*, T*, G*, and C*, E is independently selected from the group consisting of A, T, G, and C, and K is independently selected from the group consisting of A, T, G, C, A*, T*, G*, and C*, wherein n is an integer from 4 to 25 and m is an integer from 2 to 10, wherein A* is selected from the group consisting of 2-aminopurine and 2,6-diaminopurine, G* is hypoxanthine, T* is selected from the group consisting of 2-thiothymine and 2-thiouracil, and C* is selected from the group consisting of N⁴-ethylcytosine and N⁴-methylcytosine, wherein said composition includes at least one A*, one T*, one G* and one C*.

8. The compound of claim 7, wherein A* is selected from the group consisting of 2-aminopurine and 2,6-diaminopurine, T* is 2-thiothymine, G* is hypoxanthine and C* is selected from the group consisting of N⁴-ethylcytosine and N⁴-methylcytosine.

9. A composition of matter that comprises a plurality of pairs of oligonucleotides, each oligonucleotide having the formula 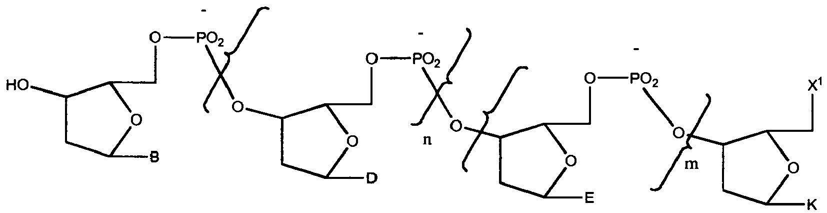 wherein X is selected from the group consisting of OH, O-phosphate, O-oligonucleotide, -NH₂, and a phosphate or an amino group linked to a biotin or fluorescent tag, B is independently selected from the group consisting of adenine, thymine, guanine, cytosine, diaminopurine, uracil, A*, T*, G*, and C*, D is independently selected from the group consisting of A*, T*, G*, and C*, E is independently selected from the group consisting of A, T, G, and C, and K is independently selected from the group consisting of A, T, G, C, A*, T*, G*, and C*, wherein n is an integer from 4 to 25 and m is an integer from 2 to 10, wherein A* is selected from the group consisting of 2-aminopurine and 2,6-diaminopurine, G* is hypoxanthine, T* is selected from the group consisting of 2-thiothymine and 2-thiouracil, and C* is selected from the group consisting of N⁴-ethylcytosine and N⁴-methylcytosine, wherein the first member of said pair is complementary to a target sequence at a portion of it, and the second member of said pair is formally the same as a segment of the same target sequence at a position between 10 and 1000 nucleotides in the 5'-direction from the portion.

10. The composition of claim 9, wherein A* is selected from the group consisting of 2-aminopurine and 2,6-diaminopurine, T* is 2-thiothymine, G* is hypoxanthine and C* is selected from the group consisting of N⁴-ethylcytosine and N⁴-methylcytosine.

11. The composition of Claim 9 wherein at least one B or one D is selected from the group consisting of N⁴-ethylcytosine and N⁴-methylcytosine.

12. The composition of Claim 9 wherein said oligonucleotides are dissolved in water at a concentration of 100 nanomolar or greater.

## Patentansprüche

1. Ein Verfahren zur Vervielfältigung eines Oligonukleotids mittels enzymatischer Matrizen-gesteuerter geprimter Polymerisation (enzymatic template-directed primed polymerization), wobei dieses Verfahren (a) das In-Kontakt-Bringen eines oder mehrerer Oligonukleotidprimerpaare in wäßriger Lösung mit dem Oligonukleotid, einer Polymerase und normalen Nukleosidtriphosphaten, und (b) das Inkubieren des Gemischs über eine vorbestimmte Zeitspanne umfasst, wobei jeder Oligonukleotidprimer in dem Paar die Formel 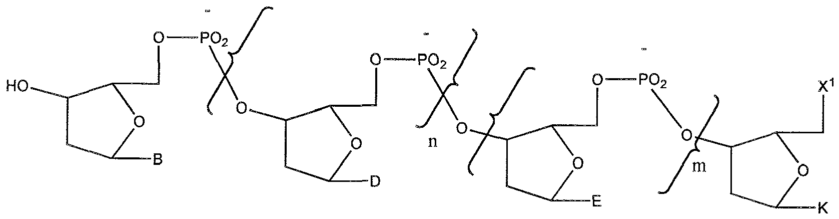 hat, wobei X aus der Gruppe bestehend aus OH, O-Phosphat, O-Oligonukleotid, -NH₂, und einem mit einem Biotin oder einer fluoreszierenden Markierung verknüpften Phosphat- oder Aminogruppe ausgewählt wird, B unabhängig aus der Gruppe bestehend aus Adenin, Thymin, Guanin, Cytosin, Diaminopurin, Uracil, A*, T*, G*, und C* ausgewählt wird, D unabhängig aus der Gruppe bestehen aus A*, T*, G* und C* ausgewählt wird, E unabhängig aus der Gruppe bestehend aus A, T, G, und C ausgewählt wird, und K unabhängig aus der Gruppe bestehend aus A, T, G, C, A*, T*, G* und C* ausgewählt wird, wobei n eine ganze Zahl von 4 bis 25 und m eine ganze Zahl von 2 bis 10 ist, wobei das erste Element des Paares komplementär zu einem Abschnitt des Oligonukleotids ist, und das zweite Element des Paares formal dasselbe ist wie ein Segment desselben Oligonukleotids, das zwischen 10 und 1.000 Nukleotide in 5'-Richtung von dem Abschnitt gelegen ist, wobei A* nicht zu der Stabilität eines Duplexes beiträgt, wenn es mit T* gepaart ist, aber dazu beiträgt, wenn es mit Thymin gepaart ist, T* nicht zu der Stabilität eines Duplexes beiträgt wenn es mit A* gepaart ist, dies aber tut wenn es mit Adenin gepaart ist, G* nicht zu der Stabilität eines Duplexes beiträgt wenn es mit C* gepaart ist, dies aber tut wenn es mit Cytosin gepaart ist, und C* nicht zu der Stabilität eines Duplexes beiträgt, wenn es mit G* gepaart ist, dies aber tut wenn es mit Guanin gepaart ist, wobei A* aus der Gruppe bestehend aus 2-Aminopurin und 2,6-Diaminopurin ausgewählt wird, T* aus der Gruppe bestehend aus 2-Thiothymin und 2-Thiouracil ausgewählt wird, G* Hypoxanthin ist und C* aus der Gruppe bestehend aus N⁴-Ethylcytosin und N⁴-Methylcytosin ausgewählt wird.

2. Das Verfahren nach Anspruch 1, wobei A* aus der Gruppe bestehend aus 2-Aminopurin und 2,6-Diaminopurin ausgewählt wird, T* 2-Thiothymin ist, G* Hypoxanthin ist und C* aus der Gruppe bestehend aus N⁴-Ethylcytosin und N⁴-Methylcytosin ausgewählt wird.

3. Das Verfahren nach Anspruch 1, wobei die Summe von m und n in den Primem mindestens 15 beträgt.

4. Das Verfahren nach Anspruch 1, wobei die D-Einheiten unabhängig voneinander aus der Gruppe bestehend aus T*, A*, G* und C* ausgewählt werden.

5. Das Verfahren nach Anspruch 1, wobei die B-Einheiten unabhängig voneinander aus der Gruppe bestehend aus Thymin, Adenin, Guanin, und Cytosin ausgewählt werden.

6. Das Verfahren nach Anspruch 1, wobei mehr als 5 dieser Primerpaare in Kontakt gebracht werden.

7. Eine Verbindung der Formel 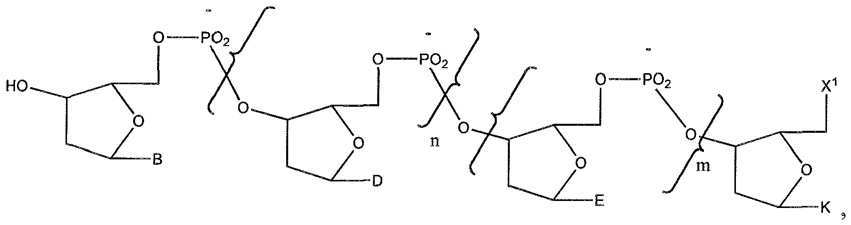 wobei X aus der Gruppe bestehend aus OH, O-Phosphat, O-Oligonukleotid, -NH₂, und einem mit einem Biotin oder einer fluoreszierenden Markierung verknüpften Phosphat- oder Aminogruppe ausgewählt wird, B unabhängig aus der Gruppe bestehend aus Adenin, Thymin, Guanin, Cytosin, Diaminopurin, Uracil, A*, T*, G*, und C* ausgewählt wird, D unabhängig aus der Gruppe bestehen aus A*, T*, G* und C* ausgewählt wird, E unabhängig aus der Gruppe bestehend aus A, T, G, und C ausgewählt wird, und K unabhängig aus der Gruppe bestehend aus A, T, G, C, A*, T*, G* und C* ausgewählt wird, wobei n eine ganze Zahl von 4 bis 25 und m eine ganze Zahl von 2 bis 10 ist, wobei A* aus der Gruppe bestehend aus 2-Aminopurin und 2,6-Diaminopurin ausgewählt wird, G* Hypoxanthin ist, T* aus der Gruppe bestehend aus 2-Thiothymin und 2-Thiouracil ausgewählt wird, und C* aus der Gruppe bestehend aus N⁴-Ethylcytosin und N⁴-Methylcytosin ausgewählt wird, wobei diese Zusammensetzung mindestens ein A*, ein T*, ein G* und ein C* einschließt.

8. Die Verbindung nach Anspruch 7, wobei A* aus der Gruppe bestehend aus 2-Aminopurin und 2,6-Diaminopurin ausgewählt wird, T* 2-Thiothymin ist, G* Hypoxanthin ist und C* aus der Gruppe bestehend aus N⁴-Ethylcytosin und N⁴-Methylcytosin ausgewählt wird.

9. Eine Stoffzusammensetzung, die eine Vielzahl von Oligonukleotidpaaren umfasst, wobei jedes Oligonukleotid die Formel 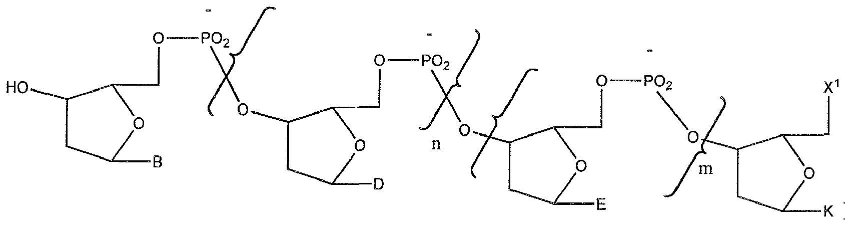 hat, wobei X aus der Gruppe bestehend aus OH, O-Phosphat, O-Oligonukleotid, -NH₂, und einem mit einem Biotin oder einer fluoreszierenden Markierung verknüpften Phosphat- oder Aminogruppe ausgewählt wird, B unabhängig aus der Gruppe bestehend aus Adenin, Thymin, Guanin, Cytosin, Diaminopurin, Uracil, A*, T*, G*, und C* ausgewählt wird, D unabhängig aus der Gruppe bestehen aus A*, T*, G* und C* ausgewählt wird, E unabhängig aus der Gruppe bestehend aus A, T, G, und C ausgewählt wird, und K unabhängig aus der Gruppe bestehend aus A, T, G, C, A*, T*, G* und C* ausgewählt wird, wobei n eine ganze Zahl von 4 bis 25 und m eine ganze Zahl von 2 bis 10 ist, wobei A* aus der Gruppe bestehend aus 2-Aminopurin und 2,6-Diaminopurin ausgewählt wird, G* Hypoxanthin ist, T* aus der Gruppe bestehend aus 2-Thiothymin und 2-Thiouracil ausgewählt wird, und C* aus der Gruppe bestehend aus N⁴-Ethyleytosin und N⁴-Methylcytosin ausgewählt wird, wobei das erste Element des Paares komplementär zu einem Abschnitt einer Zielsequenz ist, und das zweite Element des Paares formal dasselbe ist wie ein Segment derselben Zielsequenz, das zwischen 10 und 1.000 Nukleotide in 5'-Richtung von dem Abschnitt gelegen ist.

10. Die Zusammensetzung nach Anspruch 9, wobei A* aus der Gruppe bestehend aus 2-Aminopurin und 2,6-Diaminopurin ausgewählt wird, T* 2-Thiothymin ist, G* Hypoxanthin ist und C* aus der Gruppe bestehend aus N⁴-Ethylcytosin und N⁴-Methylcytosin ausgewählt wird.

11. Die Zusammensetzung nach Anspruch 9, wobei mindestens ein B oder ein D aus der Gruppe bestehend aus N⁴-Ethylcytosin und N⁴-Methylcytosin ausgewählt wird.

12. Die Zusammensetzung nach Anspruch 9, wobei diese Oligonukleotide in einer Konzentration von 100 nanomolar oder größer in Wasser aufgelöst sind.

## Revendications

1. Procédé pour amplifier un oligonucléotide par polymérisation enzymatique dirigée par une matrice, dans lequel ledit procédé comprend (a) la mise en contact dans une solution aqueuse d'une ou plusieurs paires d'amorces oligonucléotidiques avec ledit oligonucléotide, une polymérase et des nucléosides triphosphates standards, et (b) l'incubation du mélange pour une durée de temps présélectionnée, dans lequel chaque amorce oligonucléotidique dans ladite paire a la formule : 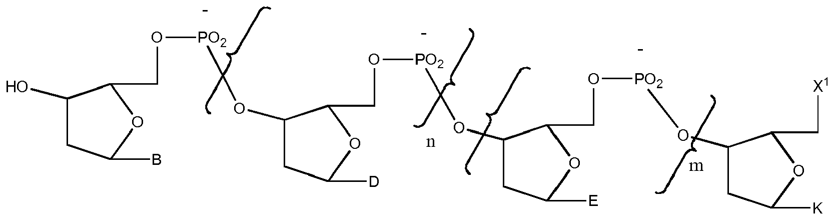 dans laquelle X est choisi parmi le groupe consistant en OH, O-phosphate, O-oligonucléotide, -NH₂, et un phosphate ou une groupe amino lié à une biotine ou une étiquette fluorescente, B est indépendamment sélectionné dans le groupe consistant en adénine, thymine, guanine, cytosine, diaminopurine, uracile, A*, T*, G* et C*, D est indépendamment sélectionné dans le groupe consistant en A*, T*, G* et C*, E est indépendamment sélectionné dans le groupe consistant en A, T, G, C, et K est indépendamment sélectionné dans le groupe consistant en A, T, G, C, A*, T*, G* et C*, dans laquelle n est un entier de 4 à 25 et m est un entier de 2 à 10, dans laquelle le premier membre de ladite paire est complémentaire dudit oligonucléotide pour une portion de celui-ci, et le second membre de ladite paire est formellement le même qu'un segment du même oligonucléotide à une position entre 10 et 1000 nucléotides dans la direction 5' de la portion, dans laquelle A* ne contribue pas à la stabilité d'un duplexe lorsqu'apparié avec T* mais y contribue lorsqu'il est apparié avec une thymine, T* ne contribue pas à la stabilité d'un duplexe lorsqu'apparié avec A* mais y contribue lorsqu'il est apparié avec une adénine, G* ne contribue pas à la stabilité d'un duplexe lorsqu'apparié avec C* mais y contribue lorsqu'il est apparié avec une cytosine, et C* ne contribue pas à la stabilité d'un duplexe lorsqu'apparié avec G* mais y contribue lorsqu'il est apparié avec une guanine, dans laquelle A* est sélectionné parmi le groupe consistant en une 2-aminopurine et une 2,6-diaminopurine, T* est sélectionné parmi le groupe consistant en une 2-thiothymine et une 2-thiouracile, G* est une hyopxanthine et C* est sélectionné parmi le groupe consistant en une N⁴-éthylcytosine et une N⁴-méthylcytosine.

2. Procédé selon la revendication 1, dans lequel A* est sélectionné parmi le groupe consistant en une 2-aminopurine et une 2,6-diaminopurine, T* est une 2-thiothymine, G* est une hyopxanthine et C* est sélectionné parmi le groupe consistant en une N⁴-éthylcytosine et une N⁴-méthylcytosine.

3. Procédé selon la revendication 1, dans lequel la somme de m et n dans ladite amorce est au moins 15.

4. Procédé selon la revendication 1, dans lequel les unités D sont indépendamment sélectionnées parmi le groupe consistant en T*, A*, G* et C*.

5. Procédé selon la revendication 1, dans lequel les unités B sont indépendamment sélectionnées parmi le groupe consistant en thymine, adénine, guanine, cytosine.

6. Procédé selon la revendication 1, dans lequel plus de 5 desdites paires d'amorces sont mises en contact.

7. Composé ayant la formule 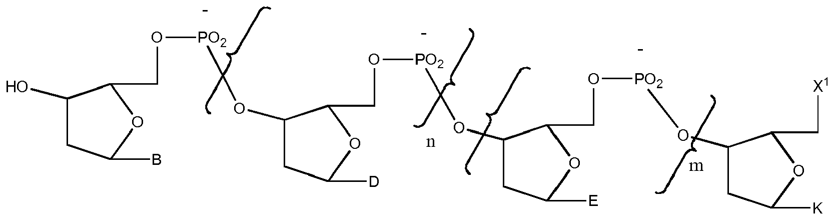 dans laquelle X est choisi parmi le groupe consistant en OH, O-phosphate, O-oligonucléotide, -NH₂, et un phosphate ou une groupe amino lié à une biotine ou une étiquette fluorescente, B est indépendamment sélectionné dans le groupe consistant en adénine, thymine, guanine, cytosine, diaminopurine, uracile, A*, T*, G* et C*, D est indépendamment sélectionné dans le groupe consistant en A*, T*, G* et C*, E est indépendamment sélectionné dans le groupe consistant en A, T, G, C, et K est indépendamment sélectionné dans le groupe consistant en A, T, G, C, A*, T*, G* et C*, dans laquelle n est un entier de 4 à 25 et m est un entier de 2 à 10, dans laquelle A* est sélectionné parmi le groupe consistant en une 2-aminopurine et une 2,6-diaminopurine, G* est une hyopxanthine, T* est sélectionné parmi le groupe consistant en une 2-thiothymine et une 2-thiouracile, et C* est sélectionné parmi le groupe consistant en une N⁴-éthylcytosine et une N⁴-méthylcytosine, dans lequel ladite composition inclut au moins un A*, un T*, un G* et un C*.

8. Composé selon la revendication 7, dans lequel A* est sélectionné parmi le groupe consistant en une 2-aminopurine et une 2,6-diaminopurine, T* est une 2-thiothymine, G* est une hyopxanthine et C* est sélectionné parmi le groupe consistant en une N⁴-éthylcytosine et une N⁴-méthylcytosine.

9. Composition qui comprend une pluralité de paires d'oligonucléotides, chaque oligonucléotide ayant la formule 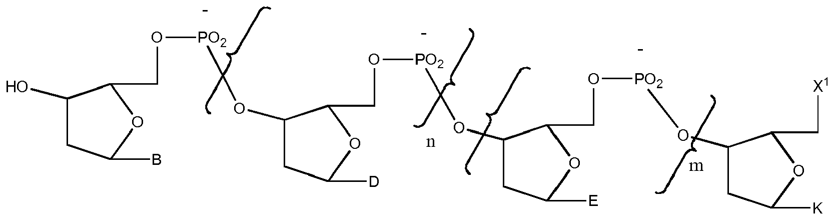 dans laquelle X est choisi parmi le groupe consistant en OH, O-phosphate, O-oligonucléotide, -NH₂, et un phosphate ou une groupe amino lié à une biotine ou une étiquette fluorescente, B est indépendamment sélectionné dans le groupe consistant en adénine, thymine, guanine, cytosine, diaminopurine, uracile, A*, T*, G* et C*, D est indépendamment sélectionné dans le groupe consistant en A*, T*, G* et C*, E est indépendamment sélectionné dans le groupe consistant en A, T, G, C, et K est indépendamment sélectionné dans le groupe consistant en A, T, G, C, A*, T*, G* et C*, dans laquelle n est un entier de 4 à 25 et m est un entier de 2 à 10, dans laquelle A* est sélectionné parmi le groupe consistant en une 2-aminopurine et une 2,6-diaminopurine, G* est une hyopxanthine, T* est sélectionné parmi le groupe consistant en une 2-thiothymine et une 2-thiouracile, et C* est sélectionné parmi le groupe consistant en une N⁴-éthylcytosine et une N⁴-méthylcytosine, dans laquelle le premier membre de ladite paire est complémentaire d'une séquence cible pour une portion de celle-ci, et le second membre de ladite paire est formellement le même qu'un segment de la même séquence cible à une position entre 10 et 1000 nucléotides dans la direction 5' de la portion.

10. Composition selon la revendication 9, dans laquelle A* est sélectionné parmi le groupe consistant en une 2-aminopurine et une 2,6-diaminopurine, T* est une 2-thiothymine, G* est une hyopxanthine et C* est sélectionné parmi le groupe consistant en une N⁴-éthylcytosine et une N⁴-méthylcytosine.

11. Composition selon la revendication 9, dans laquelle au moins un B ou un D est sélectionné parmi le groupe consistant en une N⁴-éthylcytosine et une N⁴-méthylcytosine.

12. Composition selon la revendication 9, dans laquelle lesdits oligonucléotides sont dissous dans l'eau à une concentration de 100 nanomolaire ou plus.
